# EUROPEAN PATENT SPECIFICATION

(11) **EP 3 883 594 B1**
(45) Date of publication and mention of the grant of the patent: **09.07.2025**
(21) Application number: 19806339.8
(22) Date of filing: 18.11.2019
(51) Int. Cl.: A61K 38/26, A61K 8/04, A61P 19/02, A61K 47/64, A61K 47/36, A61K 47/38

(54) **COMPOSITION AND METHODS FOR REGULATING CHONDROCYTE PROLIFERATION AND INCREASING OF CARTILAGE MATRIX PRODUCTION**
ZUSAMMENSETZUNG UND VERFAHREN ZUR REGULIERUNG DER PROLIFERATION VON CHONDROZYTEN UND ERHÖHUNG DER KNORPELMATRIXPRODUKTION
COMPOSITION ET PROCÉDÉS POUR RÉGULER LA PROLIFÉRATION DE CHONDROCYTES ET AUGMENTER LA PRODUCTION DE MATRICE CARTILAGINEUSE

(30) Priority: 19.11.2018 WO PCT/IB2018/059100
(43) Date of publication of application: 29.09.2021
(60) Divisional application: 24219712.7
(73) Proprietor: 4Moving Biotech, 59000 Lille (FR); SORBONNE UNIVERSITE, 75006 Paris (FR); ASSISTANCE PUBLIQUE - HÔPITAUX DE PARIS, 75012 Paris 12 (FR); INSTITUT NATIONAL DE LA SANTE ET DE LA RECHERCHE MEDICALE (INSERM), 75013 Paris (FR)
(72) Inventor: BERENBAUM, Francis, 91190 Gif sur Yvette (FR); RATTENBACH, Revital, 75004 Paris (FR); MARTIN, Céline, 59800 Lille (FR); MEUROT, Coralie, 59800 Lille (FR)
(74) Representative: Icosa
(86) International application number: PCT/IB2019/059889
(87) International publication number: WO 2020/104917

(56) References cited:
- WO-A1-2005/034961
- WO-A2-2008/023063
- US-A1- 2015 209 411
- JIAN CHEN ET AL: "Glucagon-like peptide-1 receptor regulates endoplasmic reticulum stress-induced apoptosis and the associated inflammatory response in chondrocytes and the progression of osteoarthritis in rat", CELL DEATH & DISEASE, vol. 9, no. 2, 1 February 2018 (2018-02-01), XP055609622, DOI: 10.1038/s41419-017-0217-y

## Description

### FIELD OF THE INVENTION

The present invention relates to novel pharmaceutical compositions comprising GLP-1 and GLP-1 analogues inducing increase in chondrocyte regeneration and a decrease in cartilage degeneration including anabolic and catabolic cytokines modulation for use in the treatment of cartilage disease.

### BACKGROUND OF THE INVENTION

Osteoarthritis (OA) is the most prevalent chronic joint disease. OA affects nearly 50% of people >65 years of age and occurs in younger individuals following joint injury. Worldwide, 250 million people suffer from OA and this disease has major economic and social impacts on patients and health care systems. OA is a disease of the whole joint, characterized by structural degradation, periarticular bone, synovial joint lining and adjacent supporting connective tissue elements. Destruction of articular cartilage is a result of chondrocytes failure to maintain the balance between synthesis and degradation of the extracellular cartilage matrix. Pro-inflammatory cytokines, such as interleukin-1β (IL-1β) produced by macrophages, monocytes, synovial cells, and chondrocytes play an important role in the development of the disease.

Glucagon Like Peptide-1 (GLP-1) is a post-translational product of the preproglucagon gene. The action of GLP-1 on pancreatic β-cell includes: increase of glucose transporter 2 expression, secretion of insulin in response to increased glucose level. Moreover, it has been shown that GLP-1 reduces the secretion of proinflammatory cytokines such as interleukin-6, tumor necrosis factor-α, and interferon-c.

GLP-1 analogues are available marketed drugs prescribed to patients for the treatment of type 2 diabetes. International Patent application WO 2017/149070 discloses some derivatives and analogues of glucagon-like peptide 1 (GLP-1), their preparation, and their pharmaceutical use. The document relates to specific derivatives and analogues of GLP-1 for use in prevention and the treatment of all forms of diabetes.

Initially, osteoarthritis has been considered to be a disease of articular cartilage, but recent research has indicated that the condition involves the entire joint.

The loss of articular cartilage has been thought to be the primary change, but a combination of cellular changes and biomechanical stresses causes several secondary changes, including subchondral bone remodeling, the formation of osteophytes, the development of bone marrow lesions, change in the synovium, joint capsule, ligaments and periarticular muscles, and meniscal tears and extrusion.

There are two patterns for the processes of cartilage growth. One of them is interstitial growth, in which cells differentiated, into chondrocytes and surrounded by the cartilage matrix proliferate through cell division. Each chondrocyte secretes a matrix, and cartilage tissue is then enlarged. The other growth pattern is appositional growth caused by the perichondrium. Cartilage tissue is covered with a perichondrium except for the articular surface of the articular cartilage. The strong perichondrium is constituted of fibroblasts, but is similar to chondrocytes in the inner layer, thus the difference between fibroblasts and chondrocytes is unclear. Perichondrium cells in the inner layer proliferate while gradually changing into circular forms, and such cells further a secrete cartilage matrix and grow outwardly.

European patent EP 2 890 390 B1 relates to an incretin hormone or an analogue thereof for use in the treatment of osteoarthritis. More particularly, the patent discloses use of GLP-1 and GLP-1 analogues, by example Liraglutide, for use in the treatment of osteoarthritis. Peptides disclosed in EP 2 890 390 B1 patent may be administered via any known administration route, including in particular systemically (parenterally, intravenously, etc.), orally, rectally, topically or subcutaneously. This patent does not disclose nor the role of GLP-1 in cartilage degradation neither demonstrates his relationship with chondrocytes.

The normal turnover of the cartilage matrix is mediated by the chondrocytes, which synthetize these components and the proteolytic enzymes responsible for their breakdown. Chondrocytes are, in turn, influenced by a number of factors, including polypeptide growth factors and cytokines, structural and physical stimuli and even the components of the matrix itself.

Osteoarthritis result from failure of chondrocytes to maintain homeostasis between anabolism and catabolism of these extracellular matrix components. It is not well-known what initiates the imbalance between the degradation and the repair of cartilage. Trauma causing a microfracture or inflammation causing a slight increase in enzymatic activity may allow the formation of wear particles, which could be then engulfed by resident macrophages. At some point in time, the production of these wear particles overwhelms the ability of the system to eliminate them and they become mediators of inflammation, stimulating the chondrocyte to release degradative enzymes. Molecules from breakdown of collagen and proteoglycan, also taken up by synovial macrophages, cause release of proinflammatory cytokines, like TNFα, IL-1 and IL-6. There is a close relationship between cytokine expression and OA. Interleukin-1 (IL-1) and tumor necrosis factor-alpha (TNF-alpha) can induce the production of interleukin-6 (IL-6) and interleukin-8 (IL-8) by synovial cells and chondrocytes. But all studies focused on synovial tissue, and not on chondrocytes.

Jian Chen et al. ("Glucagon-like peptide-1 receptor regulates endoplasmic reticulum stress-induced apoptosis and the associated inflammatory response in chondrocytes and the progression of osteoarthritis in rat" Cell death & disease, vol.9, no.2, 1 February 2018), discloses the effect of liraglutide in an osteoarthritis model in mice.

Anabolic stimulation of chondrocytes is measured in vitro by the stimulation of synthesis of proteoglycans and collagen. It has been demonstrated that cytokines such as GM-CSF, Granulocyte-Macrophage Colony Stimulating Factor (Quinetro et al., 2008 cytokine 44(3):366-72), and CXCL10/IP10 (Neidlin et al. 2018, annals of biomedical engineering, volume 46, ISSUE 2 pp 345_353) stimulate anabolic processes of chondrocytes.

There is a need to enhance chondrocytes anabolic activity including chondrocyte proliferation and decrease chondrocyte catabolic activity including cartilage matrix degradation in OA. Such improved compositions and method will have a major interest in the development of new therapeutic strategies in the treatment of Osteoarthritis.

There is also a need to enhance chondrocytes anabolic activity including chondrocyte differentiation to promote cartilage regeneration and to mitigate cartilage destruction, respectively. Such improved compositions and method will have a major interest in the development of new therapeutic strategies in the treatment of Osteoarthritis.

The present invention discloses improved pharmaceutical formulation which induces enhancement of chondrocyte anabolic processes including chondrocyte proliferation and decreases of catabolic processes including decrease of cartilage matrix loss and cartilage degradation for use in the treatment of cartilage disease.

The present invention further discloses improved pharmaceutical formulation and composition which induces enhancement of chondrocyte anabolic processes including chondrocyte differentiation for cartilage regeneration and decreases of catabolic processes including decrease of cartilage matrix loss and cartilage degradation for use in the treatment of cartilage disease.

More specifically such compositions are useful for the treatment of osteoarthritis and to prevent alleviation or reduction of joint irritation or for the reduction of worsening of existing joint inflammation.

### BRIEF SUMMARY OF THE INVENTION

The present invention relates to a composition for use in cartilage regeneration which comprises Glucagon Like Peptide-1 analogue, wherein, Glucagon Like Peptide-1 analogue is liraglutide.

In some embodiments of the present invention, the concentration of said Glucagon Like Peptide-1 is of about 0.1 nM to 625 µM.

In some embodiments of the present invention, said composition further comprises at least 5% of more weight of a pharmaceutically acceptable formulation vehicle to be used in combination.

In some embodiments of the present invention, the pharmaceutically acceptable formulation vehicle is selected from the group consisting of albumin or alpha1-acid glycoprotein.

In some embodiments of the present invention, the pharmaceutically acceptable formulation vehicle concentration is about 0.1% to about 10% (wt/wt), preferably 5% (wt/wt), of the formulation.

In some embodiments of the present invention, the pharmaceutically acceptable formulation vehicle concentration is 5% (wt/wt) of the formulation.

In some embodiments of the present invention, the pharmaceutically acceptable formulation vehicle is albumin.

In some embodiments of the present invention, the pharmaceutically acceptable formulation vehicle is alpha1-acid glycoprotein.

The present disclosure also relates on the following embodiments:
The present invention further relates to a pharmaceutical composition which induces anabolic stimulation of chondrocytes, including chondrocyte proliferation, and decreasing catabolic activity, including a decrease in cartilage matrix loss and cartilage degeneration for use in the treatment of cartilage disease.

According to a particular aspect, the present invention relates to a pharmaceutical composition which induces anabolic stimulation of chondrocytes, including chondrocyte proliferation and/or stem cell differentiation into chondrocytes for cartilage regeneration, and decreasing catabolic activity, including a decrease in cartilage matrix loss and cartilage degeneration for use in the treatment of cartilage disease.

According to a particular aspect, the disclosure relates to a pharmaceutical composition which induces anabolic stimulation of chondrocytes for use in the treatment of cartilage disease comprising Glucagon Like Peptide-1 analogue as the active ingredient therein.

According the present disclosure the Glucagon Like Peptide-1 (GLP-1) analogue is selected from the group consisting of xenatide, liraglutide, lixisenatide, albiglutide dulaglutide, semaglutide or liraglutide.

According to one aspect of the present disclosure there is provided a pharmaceutical composition which induces anabolic stimulation of chondrocytes, including chondrocyte proliferation, and/or decreasing catabolic activity, including a decrease in cartilage matrix loss for use in the treatment of cartilage disease comprising Glucagon Like Peptide-1 analogue as the active ingredient therein.

According to the present invention, the Glucagon Like Peptide-1 (GLP-1) analogue is liraglutide.

According to the present invention, the concentration of liraglutide is between 1 ng/ml and 10 mg/ml.

According to the invention, the concentration of liraglutide is between 0.1 and 10 mg/ml.

According to further features in preferred embodiments of the invention described below, the formulation of the pharmaceutical composition for use of the present invention provides a therapeutically effective amount of GLP-1 analogue and a gel comprising a polymer selected from the group consisting of non-ionic surfactant, cellulose, polyether, glucan, glycerophospholipids, polysaccharides, proteins, and combinations thereof.

According to further features in preferred embodiments of the invention described below, the formulation of the pharmaceutical composition for use of the present invention provides a therapeutically effective amount of GLP-1 analogue and an excipient comprising a polymer selected from the group consisting of non-ionic surfactant, cellulose, polyether, glucan, glycerophospholipids, polysaccharides, proteins, and combinations thereof.

Pharmaceutical formulations according to this invention can also contain one or more pharmaceutically acceptable carriers/excipients.

The present invention is not limited to gel formulation, liquid and semisolid pharmaceutical forms suitable for topical administration, such as liquid, solutions, creams, gels or transdermal patches, are preferred; in particular, forms suitable for intra-articular injection, such as liquid, solutions, and transdermal application, such as semisolid forms like creams or gels and transdermal patches. The pharmaceutical form can also consist of a form wherein some or all of the components are in a dry form, possibly lyophilized, to be reconstituted with an aqueous solution or other suitable vehicle before use.

Said formulations can be produced by methods well-known in the state of the art using known excipients such as binders, disintegrants, fillers, stabilisers, diluents and colorants. They can also include delayed- or slow-release forms made with suitable polymers known in pharmaceutical technology.

Pharmaceutically acceptable carriers/excipients such as solvents, preservatives such as antioxidants and/or chelating agents and antimicrobials, isotonicity regulators, and buffer systems are preferred for the preparation of liquid forms suitable for injectable use.

Water is preferable as solvent, possibly with co-solvents such as glycols, or polyalcohols such as ethylene glycol.

Preservatives or chelating agents may also be used, sodium edetate and sodium metabisulphite being preferred, and antimicrobials, benzyl alcohol being preferred.

Sodium chloride or mannitol are particularly preferred as isotonicity regulators.

The preferred buffer systems can be the complex of salts for the phosphate and citrate buffer, preferably in the form of sodium or potassium salts.

In the preparation of liquid forms suitable for nebulisation, pharmaceutically acceptable vehicles/excipients are preferred as solvents, with preservatives such as antioxidants and/or chelating agents and antimicrobials, isotonicity regulators, and buffer systems.

According to still further features in the described preferred embodiments of the pharmaceutical composition, the GLP-1 analogue is liraglutide and the gel comprises albumin.

According to still further features in the described preferred embodiments of the pharmaceutical composition, the GLP-1 analogue is liraglutide and comprises albumin.

According to still further features in the described preferred embodiments of the pharmaceutical composition, the GLP-1 analogue is liraglutide and comprises alpha1-acid glycoprotein (A1AGP).

According to still further features in the described preferred embodiments of the pharmaceutical composition for use according to the invention, the albumin concentration is about 0.1% to about 10% (wt/wt), preferably 5% (wt/wt), of the formulation.

According to still further features in the described preferred embodiments of the pharmaceutical composition for use according to the invention, the alpha1-acid glycoprotein (A1AGP) concentration is about 0.1% to about 10% (wt/wt), preferably 5% (wt/wt), of the formulation.

According to still further features in the described preferred embodiments, the pharmaceutical composition for comprises 1ng/ml and 10mg/ml of Liraglutide and 5% (wt/wt) of albumin.

According to still further features in the described preferred embodiments, the pharmaceutical composition for comprises 6 mg/ml of Liraglutide and 5% (wt/wt) of albumin.

According to yet another aspect of the present disclosure, the cartilage disease is selected from the group consisting of cartilage defect caused by external injuries or surgical treatment, osteochondritis dissecans, osteoarthritis, congenital cartilage disease, and cartilage injury.

The references to the methods of treatments by therapy or surgery or in vivo diagnosis methods in this description are to be interpreted as references to compounds, pharmaceutical compositions and medicaments of the present invention for use in those methods.

According to yet another aspect of the present invention there is provided a method of increasing anabolic cytokine secretion or production in chondrocytes in a patient, said method comprising administering to the patient a composition according to the invention.

According to further features in preferred embodiments, said anabolic cytokine is GMCSF and/or CXCL10/IP10.

According to yet another aspect of the present invention there is provided a method of decreasing catabolic cytokine secretion or production in chondrocytes in a patient, said method comprising administering to the patient a composition according to the invention.

According to further features in preferred embodiments said catabolic cytokine is selected from the group consisting MMP3, MMP13, PGE2, IL7, MCP1.

According to yet another aspect of the present invention, the composition is administered to the subject via intra-articular injection.

According to yet an additional aspect of the present invention there is provided a use of liraglutide for treating a cartilage disease by increasing anabolic cytokine secretion or production and lowering cartilage loss and/or repair through stimulation of chondrocyte proliferation.

According to yet an additional aspect of the present invention there is provided a use of liraglutide for treating a cartilage disease by increasing anabolic cytokine secretion or production and lowering cartilage loss and/or regeneration through stimulation of chondrocyte proliferation.

According to a particular aspect of the present invention, there is provided a use of liraglutide in the manufacture of a medicament for treating a cartilage disease by increasing chondrocyte anabolic function.

The cartilage disease is selected from the group consisting of cartilage defect caused by external injuries or surgical treatment, osteochondritis dissecans, osteoarthritis, congenital cartilage disease, and cartilage injury.

According to still an additional aspect of the present invention there is provided a use of liraglutide for treating a cartilage disease by decreasing catabolic cytokine secretion or production and lowering cartilage loss and/or repair through stimulation of chondrocyte proliferation.

According to still an additional aspect of the present invention there is provided a use of liraglutide for treating a cartilage disease by decreasing catabolic cytokine secretion or production and lowering cartilage loss and/or regeneration through stimulation of chondrocyte proliferation.

The cartilage disease is selected from the group consisting of cartilage defect caused by external injuries or surgical treatment, osteochondritis dissecans, osteoarthritis, congenital cartilage disease, and cartilage injury.

According to yet another aspect of the present invention there is provided a method of promoting the proliferation of chondrocyte cells comprising contacting the at least one chondrocyte cell with a composition according to the present invention. According to further features in preferred embodiments, the cells are mammalian cells. According to further features in preferred embodiments the cells are human cells.

According to yet another aspect of the present invention there is provided a method for treating a patient diagnosed with or at risk of developing an immunoinflammatory disorder, said method comprising administering to the patient a composition according to the invention.

According to yet another aspect of the present invention there is provided a method of treating an inflammatory pathology in a subject comprising administering to the subject a composition according to the invention. According to further features in preferred embodiments, the inflammatory pathology is an inflammatory condition occurring in the joint and joint space, and degeneration of the cartilage matrix and osteoarthritis. According to further features in preferred embodiments of the methods according to the invention, the composition is administered to the subject via intra-articular injection.

According to yet another aspect of the present invention, the composition is administered via intra-articular injection to the fat pad of the joint.

According to yet another aspect of the present invention there is provided a method of promoting cartilage matrix repair in a subject comprising administering a composition according to the invention.

According to yet another aspect of the present invention there is provided a method of promoting cartilage matrix regeneration in a subject comprising administering a composition according to the invention.

According to yet another aspect of the present invention there is provided a method of ameliorating a pro-inflammatory pathology in a subject comprising administering a composition according to the invention.

According to still another aspect of the present invention there is provided a use of liraglutide as the active ingredient in the manufacture of a pharmaceutical formulation for the alleviation or reduction of joint irritation or for the reduction of worsening of existing joint inflammation in a mammalian subject.

According to still another aspect of the present invention there is provided a use of liraglutide as the active ingredient for use in a method for the alleviation or reduction of joint irritation or for the reduction of worsening of existing joint inflammation in a mammalian subject, wherein the formulation is to be administered via intra-articular injection to the fat pad of the joint.

According to still another aspect of the present invention there is provided a use of liraglutide as the active ingredients in the manufacture of an injectable pharmaceutical formulation for the alleviation or reduction of joint irritation or for the reduction of worsening of existing joint inflammation in a mammalian subject, wherein the liraglutide is formulated together with at least a second therapeutic agent, wherein the second therapeutic agent comprises an anti-inflammatory agent, an antioxidant, a vitamin, a polyol or a combination thereof.

According to still another aspect of the present invention there is provided a composition for use as an agent for differentiating mesenchymal stem cells into chondrocytes comprising liraglutide as the active ingredient therein.

According to yet another aspect of the present invention, the concentration of liraglutide is between 1ng/ml and 10mg/ml.

According to still another aspect of the present invention there is provided a method for differentiating mesenchymal stem cells into chondrocyte, comprising the steps of :
a) Adding a composition according the present invention to a cell culture medium comprising mesenchymal stem cells;
b) Differentiating the mesenchymal stem cells into chondrocytes.

According to still another aspect of the present invention there is provided a method for differentiating mesenchymal stem cells into chondrocyte, comprising the steps of :
c) Adding a GLP-1 analogue to a cell culture medium comprising mesenchymal stem cells;
d) Differentiating the mesenchymal stem cells into chondrocytes.

According to another aspect of the present invention, the Glucagon Like Peptide-1 (GLP-1) analogue is liraglutide.

According to yet another aspect of the present invention, the concentration of liraglutide is between 0.1nM and 625 microM.

According to yet another aspect of the present invention the cell culture medium further contains MesenPRO RS growth supplement and 1% L-Glutamine.

According to still another aspect of the present invention there is provided a use of a SOX9 expression enhancing peptide in the manufacture of a medicament comprising said SOX9 expression enhancing peptide for the treatment or prevention of arthrosis wherein the SOX9 expression enhancing peptide is GLP-1 analogue which selectively targets a SOX9 gene. According to another aspect of the present invention, the GLP-1 analogue is liraglutide. According to yet another aspect of the present invention, the concentration of liraglutide is between 1ng/ml and 10mg/ml.

According to still another aspect of the present invention there is provided a use of a SOX9 expression enhancing peptide in the manufacture of a medicament comprising said SOX9 expression enhancing peptide for the treatment or prevention of inflammation wherein the SOX9 expression enhancing peptide is GLP-1 analogue which selectively targets a SOX9 gene. According to another aspect of the present invention, the GLP-1 analogue is liraglutide. According to yet another aspect of the present invention, the concentration of liraglutide is between 1ng/ml and 10mg/ml.

According to still another aspect of the present invention there is provided a pharmaceutical composition for use in the treatment or prevention of arthrosis, comprising a pharmaceutically acceptable carrier and a SOX9 expression enhancing peptide wherein the SOX9 expression enhancing peptide is a GLP-1 analogue which selectively targets a SOX9 gene. In a preferred embodiment, the SOX9 expression enhancing peptide is liraglutide which selectively targets a SOX9 gene. In another preferred embodiment of the invention, the concentration of liraglutide is between 1ng/ml and 10mg/ml.

According to still another aspect of the present invention there is provided a pharmaceutical composition for use in the treatment or prevention of inflammation, comprising a pharmaceutically acceptable carrier and a SOX9 expression enhancing peptide wherein the SOX9 expression enhancing peptide is a GLP-1 analogue which selectively targets a SOX9 gene. In a preferred embodiment, the SOX9 expression enhancing peptide is liraglutide which selectively targets a SOX9 gene. In another preferred embodiment of the invention, the concentration of liraglutide is between 1ng/ml and 10mg/ml.

According to still another aspect of the invention, the pharmaceutical composition for use in the treatment or prevention of arthrosis, comprising a pharmaceutically acceptable carrier and a SOX9 expression enhancing peptide according to the invention provides a therapeutically effective amount of liraglutide and a gel comprising a polymer selected from the group consisting of non-ionic surfactant, cellulose, polyether, glucan, glycerophospholipids, polysaccharides, proteins, and combinations thereof. In a preferred embodiment, the gel comprises albumin and wherein the albumin concentration is about 0.1% to about 10% (wt/wt), preferably 5% (wt/wt), of the formulation.

In another preferred embodiment, the gel comprises alpha1-acid glycoprotein and wherein the alpha1-acid glycoprotein concentration is about 0.1% to about 10% (wt/wt), preferably 5% (wt/wt), of the formulation.

According to still another aspect of the invention, the pharmaceutical composition for use in the treatment or prevention of inflammation, comprising a pharmaceutically acceptable carrier and a SOX9 expression enhancing peptide according to the invention provides a therapeutically effective amount of liraglutide and a gel comprising a polymer selected from the group consisting of non-ionic surfactant, cellulose, polyether, glucan, glycerophospholipids, polysaccharides, proteins, and combinations thereof. In a preferred embodiment, the gel comprises albumin and wherein the albumin concentration is about 0.1% to about 10% (wt/wt), preferably 5% (wt/wt), of the formulation.

According to another aspect of the invention of the present invention relates to a composition for use in cartilage regeneration which comprises Glucagon Like Peptide-1 analogue wherein, the Glucagon Like Peptide-1 analogue is liraglutide.

According to another aspect of the disclosure regarding the composition for use in cartilage regeneration, Glucagon Like Peptide-1 analogue is selected from the group consisting of xenatide, liraglutide, lixisenatide, albiglutide dulaglutide, semaglutide or liraglutide.

According to another aspect of the composition for use in cartilage regeneration, Glucagon Like Peptide-1 analogue is liraglutide.

According to another aspect of the composition for use in cartilage regeneration, the concentration of said Glucagon Like Peptide-1 is of about 0.1nM to 625µM.

According to another aspect, the composition for use in cartilage regeneration further comprises at least 5% of more weight of a pharmaceutically acceptable formulation vehicle to be used in combination.

According to another aspect of the composition for use in cartilage regeneration, composition for use in cartilage regeneration, the pharmaceutically acceptable formulation vehicle is selected from the group consisting of albumin or alpha1-acid glycoprotein.

According to another aspect of the composition for use in cartilage regeneration, the pharmaceutically acceptable formulation vehicle concentration is about 0.1% to about 10% (wt/wt), preferably 5% (wt/wt), of the formulation.

According to another aspect of the composition for use in cartilage regeneration, the pharmaceutically acceptable formulation vehicle concentration is 5% (wt/wt) of the formulation.

According to another aspect of the composition for use in cartilage regeneration, the pharmaceutically acceptable formulation vehicle is albumin.

According to another aspect of the composition for use in cartilage regeneration, the pharmaceutically acceptable formulation vehicle is alpha1-acid glycoprotein.

According to yet another aspect of the present invention, the composition for use in cartilage regeneration is intended to be administered orally, subcutaneously, intravenously or intra-articularly.

According to yet another aspect of the present invention, the composition for use in cartilage regeneration is administered by intra-articular injection to cartilage injury According to yet another aspect of the present invention, the composition for use in cartilage regeneration induces anabolic stimulation of chondrocytes, including chondrocyte proliferation and/or stem cell differentiation into chondrocytes.

The invention has utility where stimulation of chondrocyte proliferation or growth or chondrocyte formation from mesenchymal stem cells is viewed as desirable, including cartilage repair.

The invention therefore has utility in any application where stimulation of chondrocyte proliferation or growth is viewed as desirable, including cartilage repair and/or regeneration.

The applicants have found that increasing the effective concentration of liraglutide on OA patient chondrocytes has the effect of stimulating chondrocyte anabolic cytokines and decreasing catabolic cytokines.

The inventors demonstrated that using intra-articular injections (acute or repeated) of the pharmaceutical composition formulation and a slow release of liraglutide into the synovial liquid induces a decrease and a delay in fibrotic processes induced after cartilage damage and a real functional and histological improvement of the articular joint after joint damage.

Moreover, it was demonstrated that a specific dose regimen (i.e. several injections, separated by one week each), are necessary to produce chondrocytic proliferation with no fibrosis.

According to the present invention, the term regeneration includes chondrocyte anabolic function/proliferation in the cartilage in the absence of fibrosis leading to functional and histological improvement of the articular joint.

Using a chemically-induced OA model, the inventors demonstrated that using intra-articular injection of the pharmaceutical composition formulation and a slow release of liraglutide into the synovial liquid induces SOX9 expression.

According to its major aspects and broadly stated, the present disclosure provides a pharmaceutical composition which induces enhancement of chondrocyte proliferation, increase of cartilage repair and a decrease cartilage matrix for use in the treatment of cartilage disease which is selected from the group consisting of cartilage defect caused by external injuries or surgical treatment, osteochondritis dissecans, osteoarthritis, congenital cartilage disease, and cartilage injury comprising Glucagon Like Peptide-1 analogue as the active ingredient therein.

According to another major aspects and broadly stated, the present disclosure provides a pharmaceutical composition which induces enhancement of chondrocyte proliferation, increase of cartilage regeneration and a decrease cartilage matrix loss for use in the treatment of cartilage disease which is selected from the group consisting of cartilage defect caused by external injuries or surgical treatment, osteochondritis dissecans, osteoarthritis, congenital cartilage disease, and cartilage injury comprising Glucagon Like Peptide-1 analogue as the active ingredient therein.

### DESCRIPTION OF THE FIGURES

FIG.1: Differential expression level of calculated concentration mean of secreted cytokines in OA patient chondrocytes after treatment with several doses of Victoza^{®}.
FIG.2: Release Profile for formulations 6, 8, 14, 17, 19, 20.
FIG.3: Weight-bearing changes in percent (R/L) in Surgery induced OA during the study.FIG.4: Histology findings in Surgery induced OA during the study.
FIG.5: Dose response of formulated Liraglutide IA on knee measurements at termination.
FIG.6: Representative picture of right knee section stained with hematoxylin and eosin showing fibrous synovial chronic proliferation and tibial plate fibrosis, especially in Victoza^{®} subcutaneous injected animals.
FIG.7: Representative picture of right knee section stained with hematoxylin and eosin showing absence of fibrosis and chondrocytes nests in formulated liraglutide injected animals.
FIG.8: Long term decrease of cartilage matrix loss in formulated liraglutide injected animals compared with vehicle animals.
FIG. 9: Long term decrease of cartilage degeneration score in formulated liraglutide injected animals compared with vehicle animals.
FIG.10: Representative pictures of right knee sections stained with toluidine blue showing synovial thickening difference between formulated liraglutide injected animals compared with vehicle animals.
FIG. 11: Medial Joint capsule repair.
FIG. 12: Representative picture of right knee section stained with hematoxylin and eosin showing chondrocytes nests in group 8M.
FIG.13: Evaluation of the total number and density of chondrocyte nests.
FIG.14: Effect of Liraglutide on sphere formation process and showing positive alcian blue staining of formed chondrocytes from mesenchymal stem cells.
FIG.15: Effect of different doses of Liraglutide on Lactate dehydrogenase secretion by chondrocytes into culture medium.
FIG.16: SOX9 RTqPCR analyses on knee articular structures of monoiodoacetate-injected mice treated by liraglutide.
FIG. 17: Total joint score (histology) of injected knees of animals from group 5M treated with A1AGP vehicle and 6M treated with A1AGP-formulated Liraglutide.
FIG.18: Representative pictures of right knee sections stained with toluidine blue of animals from group 5M (A) treated with vehicle and 6M (B) treated with A1AGP-formulated Liraglutide.

### DETAILED DESCRIPTION OF THE INVENTION

The present invention discloses specific composition for inducing enhancement of chondrocyte differentiation and chondrocyte proliferation and increase of cartilage matrix production for use in the treatment of cartilage disease and osteoarthritis. Such compositions can be directly administered to the affected joint, preferably by direct injection into the closed cavity of the joint (intraarticular injection).

Before describing the present invention in detail, it is to be understood that unless otherwise indicated this invention is not limited to specific materials or manufacturing processes, as such may vary. It is also to be understood that the terminology used herein is for the purpose of describing particular embodiments only and is not intended to be limiting.

The articles "a" and "an" are used herein to refer to one or to more than one (i.e., to at least one) of the grammatical object of the article. By way of example, "an element" means one element or more than one element or "a protein" means more than one protein.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. Therefore, about 50% means in the range of 45%-55%. Numerical ranges recited herein by endpoints include all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about."

The term, "chondrocyte" refers to cells isolated from cartilage.

The term, "cartilage" or "articular cartilage" or "cartilage matrix" refers to elastic, translucent connective tissue in mammals, including human and other species. Cartilage is composed predominantly of chondrocytes, type II collagen, small amounts of other collagen types, other noncollagenous proteins, proteoglycans and water, and is usually surrounded by a perichondrium, made up of fibroblasts, in a matrix of type I and type II collagen as well as other proteoglycans. Although most cartilage becomes bone upon maturation, some cartilage remains in its original form in locations such as the nose, ears, knees, and other joints. The cartilage has no blood or nerve supply and chondrocytes are the only type of cell in this tissue.

The terms "active agent," "active excipient", "active ingredient", "pharmacologically active excipient" are used interchangeably herein to refer to a chemical material or compound that induces a desired pharmacological, physiological effect, and include agents that are therapeutically effective, prophylactically effective. The terms also encompass pharmaceutically acceptable, pharmacologically active derivatives and analogs of those active agents specifically mentioned herein, including, but not limited to, salts, esters, amides, prodrugs, active metabolites, inclusion complexes, analogs, and the like.

The term "effective amount" or "a therapeutically effective amount" of a pharmacologically active agent or active excipient is intended to mean a nontoxic but sufficient amount of the agent or excipient to provide the desired therapeutic effect. The amount that is "effective" will vary from subject to subject. Thus, it is not always possible to specify an exact "effective amount." However, an appropriate "effective" amount in any individual case may be determined by one of ordinary skill in the art using routine experimentation. Furthermore, the exact "effective" amount of an active agent incorporated into a composition or dosage form of the invention is not critical, so long as the concentration is within a range sufficient to permit ready application of the formulation so as to deliver an amount of the active agent that is within a therapeutically effective range.

Preferred routes of administration are local, including topical, or application to an injured cartilage site or a site of (surgical) intervention at or near to cartilage, preferably in the form of a fluid, in a hydrogel or collagen matrix or an artificial scaffold (matrix).

The present invention also discloses pharmaceutical compositions comprising the compounds of the present invention. More particularly, such compounds can be formulated as pharmaceutical compositions using standard pharmaceutically acceptable carriers, fillers, solubilizing agents and stabilizers known to those skilled in the art.

The invention disclosesthe preparation and use of pharmaceutical compositions comprising a compound useful for treatment of the diseases disclosed herein as an active ingredient. Such a pharmaceutical composition may consist of the active ingredient alone, in a form suitable for administration to a subject, or the pharmaceutical composition may comprise the active ingredient and one or more pharmaceutically acceptable carriers, one or more additional ingredients, or some combination of these.

The active ingredient may be present in the pharmaceutical composition in the form of a physiologically acceptable ester or salt, such as in combination with a physiologically acceptable cation or anion, as is well known in the art.

As used herein, the term "physiologically acceptable" ester or salt means an ester or salt form of the active ingredient which is compatible with any other ingredients of the pharmaceutical composition, which is not deleterious to the subject to which the composition is to be administered.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered.

By way of example, the composition may comprise between 0.1 % and 100% (w/w) active ingredient. In addition to the active ingredient, a pharmaceutical composition of the invention may further comprise one or more additional pharmaceutically active agents.

As used herein, "additional ingredients" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials.

The composition may also comprise one or more substances used in the treatment of osteoarthritis, in particular one or more inhibitors of the dipeptidyl peptidase IV enzyme, preferably chosen from the group consisting of sitagliptin, saxagliptin, vildagliptin, alogliptin and linagliptin, or other substances such as analgesics, non-steroidal anti-inflammatories, steroidal anti-inflammatories and slow-acting anti-arthritic agents. analgesics comprising paracetamol; acetylsalicylic acid, lysine acetylsalicylate, phenylbutazone, sulindac, diclofenac potassium or sodium, aceclofenac, tiaprofenic acid, ibuprofen, ketoprofen, alminoprofen, fenoprofen, naproxen, flurbiprofen, indomethacin, mefenamic acid, niflumic acid, tenoxicam, meloxicam, piroxicam, and selective cyclooxygenase-2 inhibitors such as celecoxib and etoricoxib, betamethasone, dexamethasone, prednisolone, prednisone, tixocortol or triamcinolone; chondroitin, chondroitin sulphate (Structum, Chondrosulf), glucosamine or glucosamine sulphate, diacerein (Art 50, Zondar), or unsaponifiable extracts of avocado and soya (piascledine).

Other "additional ingredients" which may be included in the pharmaceutical compositions of the invention are known in the art.

The formulation of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi- dose unit.

It will be understood by the skilled artisan that such pharmaceutical compositions are generally suitable for administration to animals of all sorts. In a preferred embodiment, the subject to be treated, or patient, is an animal, preferably a mammal. According to one embodiment, the subject to be treated is an animal selected from the group consisting of a dog, a cat, a horse, a cow, a sheep, a pig and a non-human primate.

According to one preferred embodiment, the subject to be treated is a human, preferably an adult, and particularly preferably an adult over the age of 50.

The composition according to the invention may be administered via any known administration route, including in particular systemically (parenterally, intravenously, etc.), orally, rectally, topically or subcutaneously. According to one preferred embodiment, the composition may also be administered by intra-articular injection, preferably into the arthritic joint. In this case, it may be administered in combination with other locally acting substances such as hyaluronic acid, albumin, alpha-1 glycoprotein, or analgesic substances.

The compound may be administered to an animal as frequently as several times daily, or it may be administered less frequently, such as once a day, once a week, once every two weeks, once a month, or even less frequently, such as once every several months or even once a year or less.

The frequency of the dose will be readily apparent to the skilled artisan and will depend upon any number of factors, such as, but not limited to, the type and severity of the condition or disease being treated, the type and age of the animal, etc.

A pharmaceutical composition of the invention may be prepared, packaged, or sold in bulk, as a single unit dose, or as a plurality of single unit doses.

The term "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The invention is also directed to methods of administering the compounds of the invention to a subject. In one embodiment, the invention provides a method of treating a subject by administering compounds identified using the methods of the invention.

As used in this document, the term "treatment" or "therapy" refers to any action which makes it possible to reduce, suppress or delay the symptoms associated with a pathological condition. It comprises both a curative treatment and a prophylactic treatment for a disease. A curative treatment is defined by a treatment resulting in a cure or a treatment which relieves, improves and/or eliminates, reduces and/or stabilizes the symptoms of a disease or the suffering that it causes. A prophylactic treatment comprises both a treatment resulting in the prevention of a disease and a treatment which reduces and/or delays the incidence of a disease or the risk of it occurring.

In particular, in the context of the present invention, the term "treatment" refers more particularly to the inhibition or the slowing down of the arthritic destruction of cartilage.

The term "therapeutically effective dose" as used herein refers to the amount required to observe a therapeutic or preventive activity on the osteoarthritis, in particular the amount required to observe an inhibition or a slowing down of the arthritic cartilage destruction. The amount of peptide to be administered and the duration of the treatment are evaluated by those skilled in the art according to the physiological condition of the subject to be treated, the nature of the arthritic joint(s) to be treated,

In some embodiment, the composition disclosed here can also be used in the treatment of a primary osteoarthritis (without anatomical or traumatic cause) or secondary osteoarthritis. The osteoarthritis treated may affect any joint, in particular the joints of the hip (coxarthrosis), the knee (gonarthrosis), the ankle, the foot, the hand, the wrist, the elbow, the shoulder or the rachis, preferably the joints of the hip, the knee, the hand and the rachis.

The present invention also discloses the use of liraglutide as the active ingredient in the manufacture of a pharmaceutical formulation for the alleviation or reduction of joint irritation or for the reduction of worsening of existing joint inflammation in a mammalian subject.

The present invention also discloses a method for increase the chondrocyte proliferation in a patient, said method comprising the administration to said patient of a therapeutically effective dose of a composition consisting of liraglutide between 1ng/ml to 10 mg/ml and albumin between 0.1% to 10%.

As discloses here, a method of treating inflammatory pathology a subject in need of such treatment is provided. The method comprises administering a pharmaceutical composition comprising at least one compound of the present invention to a subject in need thereof. Compounds identified by the methods of the invention can be administered with known compounds or other medications as well.

The characteristics and advantages of the invention will emerge more clearly on reading the following examples given by way of non-limiting illustration.

### EXAMPLE 1: Effect of VICTOZA^{®} ON cytokine release in OA PATIENTS chondrocytes

The study aimed to evaluate the effect of Victoza^{®}, a GLP-1 analogue, on the release of inflammatory modulators from IL-1 β-stimulated human chondrocytes isolated from cartilage of osteoarthritic patients.

### MATERIAL AND METHODS

Test Item: Victoza^{®} (Novo Nordisk).

Reference Items: Water for injection.

Material for cell culture: DMEM, Fetal Bovine Serum, Penicillin Streptomycin, Phosphate Buffered Saline, Liberase Blendzyme 3, IL-1β.

Test system: MMP3 ELISA kit, MMP13 ELISA kit, PGE2 ELISA kit, Cytokine 30-Plex Panel Assay. ELISA and multiplex assays were performed according to manufacturer's instructions.

Formulation of Test and Reference Items preparation: Victoza stock solution is at 6 mg/ml. Molecular weight is 3751.202 g/mol.

For each patient, 4 ml of medium containing Victoza^{®} at 5, 25, 50, 125 and 625 nM were prepared. To this aim, solutions at 5, 25, 50, 125 and 625 µM were prepared as following:
5 µM (18.756 µg/ml): 1.56 µl of stock solution at 6 mg/ml qsp 500 µl sterile water
25 µM (93.78 µg/ml): 1.56 µl of stock solution at 6 mg/ml qsp 100 µl sterile water
50 µM (187.56 µg/ml): 1.56 µl of stock solution at 6 mg/ml qsp 50 µl sterile water
125 µM (468.2 µg/ml): 1.56 µl of stock solution at 6 mg/ml qsp 20 µl sterile water
625 µM (2344.5 µg/ml): 1.95 µl of stock solution at 6 mg/ml qsp 5 µl sterile water.

These solutions were prepared for each patient and were diluted 1:1000 in culture medium (4 µl in 4 ml) to reach final concentrations. Vehicle consisted of 4 µl of sterile water in 4 ml of culture medium.

Formulation of liberase solution: stock solution of liberase at 26 U/ml was prepared in DMEM containing 1% P/S and 2% Glutamine and stored at -20°C. For the 2 first steps of digestion, solution at 0.52 U/ml was prepared extemporaneously in DMEM. For the last step of digestion, solution at 0.13 U/ml was prepared by diluting the 0.52 U/ml solution at 1:4.

Formulation of cell culture medium: 15% FBS, 2% L-Glutamine, 1% Penicillin/Streptomycin in DMEM with 4.5 g/L of glucose.

### EXPERIMENTAL DESIGN AND CONDITIONS

Cartilages were isolated from four patients with osteoarthritis undergoing knee surgery with implementation of a prosthesis at Hospital Saint Antoine. Isolation, seeding, culture and activation of chondrocytes and sample preparation were performed. ELISA and multiplex analysis were performed afterwards.

Day of isolation of human articular cartilage was considered as "day 1" and study termination as "day 14".

Isolation of chondrocytes from human cartilage: cartilages were isolated from patients with osteoarthritis undergoing knee surgery with implementation of a prosthesis. Cartilage from one patient was processed at a time. Freshly isolated cartilages were cut in pieces of 2-3 mm diameter, placed in a 50 ml tube and rinsed with PBS. The pieces of cartilage were incubated for 45 minutes in 40 ml of liberase at 0.52 U/ml in DMEM. After 45 minutes, liberase was removed and a new solution of liberase at 0.52 U/ml was added for 45 minutes. Then, the solution was removed and the pieces of cartilage were incubated overnight in 40 ml of liberase (0.13 U/ml).

Seeding of chondrocytes: 16 hours following chondrocytes isolation, solution was pipetted up and down to homogenize the cells. Thereafter, the solution was filtered through a 100 µm cell strainer. Filtered solution was centrifuged at 1600 rpm for 6 minutes at room temperature. Pellet was resuspended in 15 ml of complete medium (DMEM + 15% FBS+ 2% glutamine + 1% P/S). Cells were counted by hemocytometer and seeded on 12-well culture plates at density of 200000-250000 cells per well. The cultures were incubated under sterile conditions (37°C, 5% CO2).

Culture of chondrocytes: 48 hours following seeding, medium was replaced with a fresh medium. Thereafter, medium was renewed every 2 days until confluence (at day 12-13). At confluence, 24 hours before treatments, medium was replaced by medium without FBS and with 0.1% BSA.

The next day, chondrocytes were pre-incubated with 5 doses of Victoza^{®} (5 nM, 25 nM, 50 nM, 125 nM, 625 nM) or vehicle for 2 hours and then stimulated with IL-1β (5 ng/ml) for 24 hours according to study design (Table 1) and schedule (Table 2).

**Table 1. Study design.**

| Group | Treatment | Time |
|---|---|---|
| 1 | Vehicle with IL-1β | 24H |
| 2 | Victoza 5 nM with IL-1β | |
| 3 | Victoza 25 nM with IL-1β | |
| 4 | Victoza 50 nM with IL-1β | |
| 5 | Victoza 125 nM with IL-1β | |
| 6 | Victoza 625 nM with IL-1β | |

**Table 2. Study schedule.**

| Study day* | Procedure |
|---|---|
| 1 | Isolation of cartilage and chondrocytes |
| 2 | Seeding and culture of chondrocytes |
| 12 | Replacement by medium without FBS |
| 13 | Pretreatment with Victoza and activation with IL-1β |
| 14 | Recuperation of cell supernatants |

### TESTS AND EVALUATIONS: ELISA AND MULTIPLEX ASSAYS

Preparation of samples: at termination of each study, culture medium was collected, centrifuged and supernatant were frozen. Samples were shipped in dry ice in the test facility and store at -80°C upon reception until analysis.

### Detection assays.

PGE2 assay: the assays were based on competition between free PGE2 and a PGE2-acetylcholinesterase conjugate (PGE2 Tracer) for a limited amount of PGE2 monoclonal antibody. The concentration of PGE2 Tracer was held constant whereas free PGE2 varied in each sample. The amount of PGE2 Tracer which bound the monoclonal antibody was inversely proportional to the amount of free PGE2 in the sample. The enzymatic reaction involved acetylcholinesterase substrate so the color was little intense in the PGE2 high-concentrated samples and very intense in PGE2 low-concentrated samples.

According to manufacturer's instructions, concentrations were calculated after determination of %B/B0, where B0 represent the absorbance obtained from the reading of the wells where the maximum amount of the PGE2 Tracer is bound (in the absence of free PGE2) and B the absorbance obtained for each standard or sample well. In order to obtain accurate results, supernatants from IL-1β treated wells were diluted 1:1000 and others were diluted 1:500 before proceeding with the test. The range of measurement of PGE2 was 7.8 to 1000 pg/ml.

MMP3 assay: The assay was based on a classical sandwich ELISA coupled with colorimetric peroxidase system detection. In order to obtain accurate results, supernatants from IL-1β treated wells were diluted 1:1000 and others were diluted 1:500 before proceeding with the test. The range of measurement of MMP3 was 0.156 to 10 ng/ml.

MMP13 assay: The assay was based on a classical sandwich ELISA coupled with colorimetric biotin-streptavidin system detection. In order to obtain accurate results, supernatants from IL-1β treated wells were diluted 1:100 and others were diluted 1:10 before proceeding with the test. The range of measurement of MMP13 was 8.23 to 6000 pg/ml.

Multiplex assay: The Luminex technology is based on the coupling of ELISA sandwich technique and mix of fluorescent polystyrene beads which represent the solid phase for detection. Each bead is coupled with a specific antibody and a different fluorochrome. This assay allows to quantify various cytokines in the same sample with minimal volume utilization. The Human Cytokine Magnetic 30-plex from Life Technologies was used to quantify EGF, Eotaxin, FGF basic, GCSF, GMCSF, HGF, IFN-α, IFN-γ, IL-1RA, IL-1β, IL-2, IL-2R, IL-4, IL-5, IL-6, IL-7, IL-8, IL-10, IL-12 (p40/p70), IL-13, IL-15, IL-17, IP-10, MCP1, MIG, MIP1, MIP1β, RANTES, TNF-α and VEGF.

The samples were assayed without dilution according to manufacturer's instructions.

At termination of incubation, culture medium was collected, centrifuged, then ELISA (MMP3 ELISA kit, MMP13 ELISA kit, PGE2 ELISA kit and Cytokine 30-Plex Panel Assay) assays were performed on the supernatant.

### RESULTS

Concentration of each cytokine was calculated according to manufacturer instructions.

A basal inflammatory profile for each patient was evaluated by cumulating calculated concentration mean for each detected cytokine.

The results show that the basal inflammatory profile was highly variable including two patients with high concentration of inflammatory cytokines and two patients with lower concentration of cytokines.

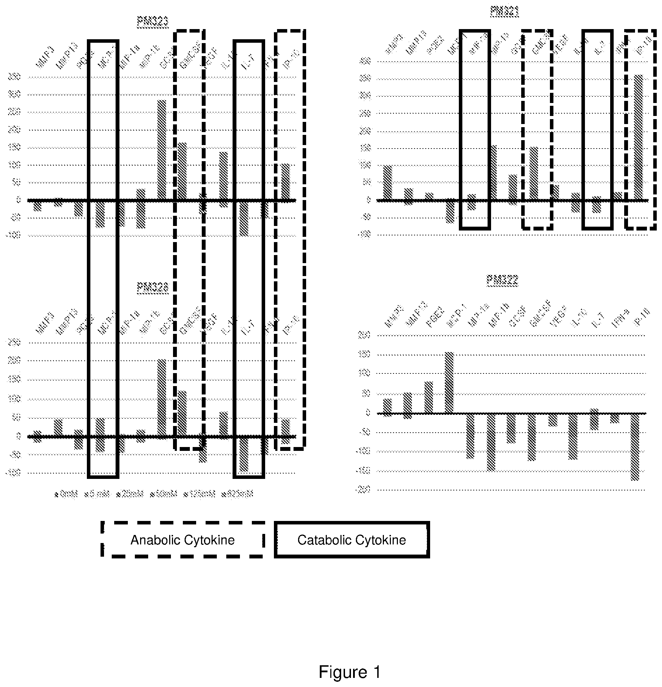

A Victoza^{®} response inflammatory profile for each patient was evaluated by comparing the ratio for each cytokine calculated concentration mean before and after Victoza^{®} treatment. The general results show a different response per patient. However, a comparable response to Victoza^{®} emerges for four cytokines in three out of four patients' chondrocytes treated with Victoza^{®} in IL-1β context; GMCSF and CXCL10/IP-10 (anabolic cytokines) calculated concentration mean, were increased while IL7, MCP1 (catabolic cytokines) were decreased (Figure 1).

### CONCLUSION

The inventors demonstrated different response profile in each of the four patients studied. Liraglutide increases some anabolic cytokine secretion and decrease some catabolic cytokine secretion in chondrocytes from OA patients.

### EXAMPLE 2: PREPARATION OF INTRAARTICULAR FORMULATIONS OF LIRAGLUTIDE WITH PROLONGED RELEASING - DETERMINATION OF RELEASE PROFILES

The inventors tested 20 different formulations of viscous hydrogels with Liraglutide for intraarticular injection and to determine the release profile into artificial synovial fluid of each formulation in order to choose the three best formulations for further in vivo preclinical studies

### MATERIALS AND METHODS

Test material: Liraglutide.
Materials for hydrogels formulations
Dextran 70 EP (70 kDa),
Poloxamer 407: Kolliphor^{®} P 407, Oxyethylene 71,5-74,9%,
Polyethylene Glycol (PEG) 3350
Alginic acid sodium salt,
(Hydroxypropyl)methyl cellulose (HPMC), viscosity 2,600-5,600 cP, 2 % in H2O (20 °C) (lit.),
Albumin bovine Fraction V, pH 7.0, Mr 67.000,00,
Polysorbate 80: Tween^{®} 80
Lecithin from soybean,
Polyethylene Glycol (PEG) 400,
Chitosan 95/500, high viscosity,
Sodium Hyaluronate 1.9MDa,
PBS pH 7.4
Materials for artificial synovial fluid formulation
Sodium Hyaluronate 1.9MDa, Wellcos - Markus Grauel
Albumin bovine Fraction V, pH 7.0, Mr 67.000,00, SERVA
γ-Globulin bovine, Mr 150.000,00, SERVA
PBS pH 7.4, Panreac AppliChem
Test system for determination of release profile
A semi-permeable membrane was used: Dialysis tubing visking, cellulose, thick. 0.023 mm, MWCO 12-14 kDa
ELISA kit for determination of liraglutide concentration
Artificial synovial fluid formulation: to formulates the artificial synovial fluid, 3.5 mg of sodium hyaluronate, 9 mg of albumin and 3.5 mg of γ-globulin were resuspended for every 1 ml in PBS pH 7.4. The volume of artificial synovial fluid prepared was of 10 ml for each experiment.

The composition of artificial synovial fluid was chosen on the basis of many information sources, e.g. Biological Performance of Materials: Fundamentals of Biocompatibility. Fourth Edition, Jonathan Black, CRC Press, 20 gru 2005; Synovial Fluid Composition and Functions. Dr Arun Pal Singh, http://boneandspine.com/synovial-fluid/; Concentration of Hyaluronic Acid in Synovial Fluid. Barry Decker et al. Clinical Chemistry 1959, 5(5):465-469.

Liraglutide formulation for stability study: for stability study, 1 mg of Liraglutide in 1.02 ml of PBS was mixed with 10 ml of artificial synovial fluid.

### TEST PROCEDURE

Release profiles determination: for each prepared formulation, the release profile was determined. At day 0, Liraglutide hydrogels formulations (1 mg/ml - 1.02 ml) were placed into a semi-permeable membrane test system bathed into 10 ml artificial synovial fluid. The released molecules passed through the membrane into the artificial synovial fluid at 37°C. This membrane should allow free diffusion of monomers of Liraglutide but it is a physical barrier for oligomers of Liraglutide and the hydrogel formulation. Samples of artificial synovial fluid (0.2 ml each) were collected after 1, 2, 4, 7, 10 and 14 days. Taken samples were replaced with fresh artificial synovial fluid to maintain the same volume of fluid outside the membrane. Samples of artificial synovial fluid were stored at 2-8°C until end of the study for further ELISA analysis to determine Liraglutide concentration.

Stability study: to determine the stability of Liraglutide in conditions of the study, a solution of Liraglutide in artificial synovial fluid was prepared in the appropriate concentration and treated in the same way as Liraglutide hydrogel formulations.

ELISA procedure: the quantification of Liraglutide concentration was performed with an ELISA kit ELISA procedure was performed according to recommendations of kit's provider with one exception. The standards for calibration curve that were used had higher concentrations than suggested and were in a range 0.977-1000ng/ml. Samples were diluted with EIA buffer. Standards and controls were prepared from one stock solution of Liraglutide. Analysis was performed on several EIA plates. On all plates the same standard and control solutions were used. The expected concentrations of Liraglutide in samples after dilution were in a range 5-227 ng/ml. Three quality controls were used with concentrations covering expected concentration range of samples: 15, 100, and 200 ng/ml. Each point was measured in duplicate. The absorbance was measured by a 96-well plate reader at 450 nm.

### EXPERIMENTAL DESIGN AND CONDITIONS

Duration of the Experimental Period: day of formulations preparation and membrane loading was considered as "Day 0" and study termination as "Day 14".

Group design and study schedule: 20 different hydrogels formulations containing 1 mg/ml of Liraglutide were tested according to study design (Table 3) and study schedule (Table 4). Three classes of formulations were included:
standard formulation (1,2,3,4,5,7,8,10,11,12,13,14,15,16),
with potential active excipient formulation (17,18,19,20)
and albumin-based formulation 6,9).

**Table 3. Study design.**

| Formulation No. | Hydrogels composition (in PBS pH 7.4)* + 1 mg/ml liraglutide |
|---|---|
| | * Except for formulation No.17 (chitosan) which was dissolved in pH 4 and then solution was adjusted to pH 7,4 with sodium hydroxide |
| 1 | Dextran |
| 2 | Poloxamer 407 |
| 3 | Polyethylene glycol (PEG) 3350 |
| 4 | Alginate |
| 5 | Hydroxypropylmethylcellulose (HPMC) |
| 6 | Albumin |
| 7 | PEG 3350, polysorbate 80 |
| 8 | HPMC, polysorbate 80 |
| 9 | Albumin, polysorbate 80 |
| 10 | Dextran, polysorbate 80 |
| 11 | Dextran, lecithin |
| 12 | Poloxamer 407, lecithin |
| 13 | Poloxamer 407, polysorbate 80 |
| 14 | Poloxamer 407, polysorbate 80 |
| 15 | Hydroxypropylmethylcellulose (HPMC) |
| 16 | Poloxamer 407, PEG 400, HPMC |
| 17 | Chitosan |
| 18 | Hyaluronate sodium |
| 19 | Hyaluronate sodium, PEG 3350 |
| 20 | Hyaluronate sodium, poloxamer 407 |

**Table 4. Study schedule.**

| Procedure | Study day | | | | | | |
|---|---|---|---|---|---|---|---|
| | 0 | 1 | 2 | 4 | 7 | 10 | 14 |
| Formulations | √ | | | | | | |
| Membrane loading | √ | | | | | | |
| Artificial synovial fluid samples collection | | √ | √ | √ | √ | √ | √ |
| Termination | | | | | | | √ |

### RESULTS

The analyses of Liraglutide were performed accurately and provided reliable results. ELISA raw data including calibration curves are presented in Appendix II. On all plates, two of three quality controls met acceptance criterion i.e. fell within a range ± 20% of theoretical concentration. The acceptance criteria were accepted after EMA's Guideline on bioanalytical method validation (EMEA/CHMP/EWP/192217/2009 Rev. 1 Corr. 2**).

Liraglutide concentrations: samples from artificial synovial fluid were diluted 1:400 before proceeding with the ELISA staining. All collected samples from the first five time points were analyzed. Additionally, also eight samples from the last time point were analyzed based on the expectations and consistence of formulations at the beginning of the study.

Determination of release profiles: results as percentages of maximum expected Liraglutide concentration for all hydrogels formulations were calculated. The amounts of Liraglutide taken from the experiment with analytical samples were not included into calculation. The expected Liraglutide concentration into the artificial synovial fluid (if all liraglutide was released from the semi-permeable membrane) is 90.74 µg/ml (which represents 100%).

Calculated percentage of the best formulations are presented below (Table 5) of the six best formulations are presented below.

**Table 5.**

| Formulation No. | Time point [days] | | | | | |
|---|---|---|---|---|---|---|
| | 1 | 2 | 4 | 7 | 10 | 14 |
| 6 | 64% | 59% | 42% | 28% | 15% | ND |
| 8 | 23% | 28% | 24% | 8% | 6% | 1% |
| 14 | 69% | 88% | 75% | 71% | 47% | 28% |
| 17 | 74% | 44% | 41% | 56% | 68% | ND |
| 19 | 40% | 32% | 22% | 2% | 1% | ND |
| 20 | 6% | 7% | 11% | 7% | 1% | ND |

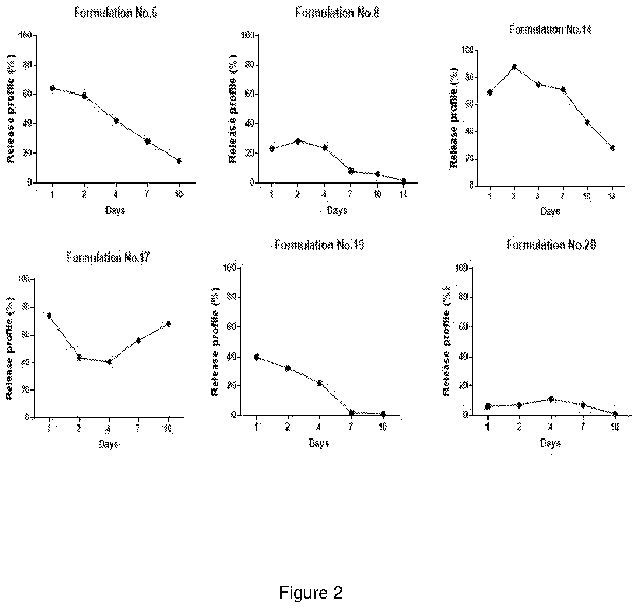

The performed experiment with the aim to determine release profiles of 20 different formulations containing Liraglutide has shown diversity among tested formulations. From the original 20 tested formulations, 6 provided Liraglutide concentration higher than 1% after ten days. These are formulations 6, 8, 14, 17, 19 and 20 with the release profile shown in figure 2.

### EXAMPLE 3: EFFICACY STUDY OF THREE LIRAGLUTIDE BASED-FORMULATIONS UTILIZING OSTEOARTHRITIS SURGICALLY INDUCED MODEL IN RATS

The purpose of the study was to evaluate 3 Liraglutide-based formulations efficacy utilizing a surgically-induced model of osteoarthritis in rats.

### MATERIALS AND METHODS

Test Item: Liraglutide
Vehicle: PBS
Formulations of Liraglutide: Three viscous hydrogels formulations were tested:
Formulation 6: High release
Formulation 8: medium release
Formulation 20: low release

### Non-formulated Liraglutide corresponds to Liraglutide which was dissolved in PBS.

Formulations were prepared on the day of the treatment for each of the three cycles. For each formulation, 4mg of Liraglutide were dissolved in 2ml of formulation solutions or PBS (for non-formulated Liraglutide) to reach 0.18mg/kg dose level in 25µl for intra-articular injection. Assuming a mean BW of 280 g, the dose administered for each rat was 50µg. Liraglutide was used for animals dosing within one hour after preparation.

### EXPERIMENTAL MODEL

Animal species / Strain: Rat / Sprague Dawley (SD)
Gender / Number / Body weight average: Male / 60 / 6-8 weeks at study initiation
Diet: Animals were fed ad libitum a commercial rodent diet.

### EXPERIMENTAL DESIGN AND CONDITIONS

Rats were allocated into one of the five stratified study groups according to the body weight.

OA induction by medial ligament transection (MLT) procedure followed by resection of medial menisci (MMx).

Anesthesia was induced for each rat by a chamber induction technique using inhalation anesthesia (Isoflurane at 4.0%). During surgery, the animal was maintained with Isoflurane at a level between 1.5 and 2.5% with an oxygen flow rate of 1-2 liters/minute. Ophthalmic ointment was applied to the eyes to prevent drying of the tissue during the anesthetic period. After induction of anesthesia, the right leg skin surface was clipped free of hair using electric animal clippers. After shaving the knee joint, the skin was disinfected with iodine and a para patellar skin incision was made on the medial side of the joint. An incision on the medial side of the joint space was made. The medial ligament was transected and the medial meniscus was resected using a microsurgical knife. The wound was closed with vicryl 5/0 braided absorbable suture. All operation procedures were performed using a surgical microscope. Group Allocation is show in Table 6 and study timeline in Table 7.

**Table 6. Group allocation.**

| Group | Treatment | OA induction | Dose Level (mg/kg) | Dose volume (µl) | Route of administ ration |
|---|---|---|---|---|---|
| 1M (1,3,4,21,22,23, 24,41,42,43,44) | Vehicle (PBS) | √ | NA | 25 | IA |
| 2M (5,6,7,8,25,26, 27,28,45,46,47,4 8) | Non-formulated Liraglutide | √ | 0.18 | 25 | IA |
| 3M (9,10,11,12,29, 30,31,32,49,50,5 1,52) | Liraglutide (formulation-6) | | 0.18 | 25 | IA |
| 4M (13,14,15,16,33, 34, 35,36,53,54,55,5 6) | Liraglutide (formulation-8) | √ | 0.18 | 25 | IA |
| 5M (17,18,19,20,37, 38, 39,40,57,58,59,6 0) | Liraglutide (formulation-20) | √ | 0.18 | 25 | IA |

**Table 7. Study timeline.**

| Study Day/week | Procedure | Remarks |
|---|---|---|
| Once a week | Body weight | |
| Day 1 | OA induction | |
| Day 7 | Treatment with Liraglutide | Single IA administration |
| Days -1, 14, 28 and 35 | Von Frey test | |
| Days -1, 14, 28 and 35 | Weight bearing test | |
| Day 36 | Termination | Right knee collection and fixation |
| | | Left knee collection and fixation |

### TESTS AND EVALUATION

Weight-bearing changes in the rats with OA were measured using an incapacitance tester. Postural imbalance, which reportedly indicates a change in the pain threshold and weight distribution of the limbs, is decreased. Each rat was placed so that each hind paw rested on a separate force plate on the incapacitance apparatus, and the weight borne by each hind limb was measured for 5 s. The ratio of the weight borne by the right to left hind limb is calculated. The mean of 5 consecutive measurements for each rat was recorded. Weight bearing function Iincapacitance test) was performed at baseline (Day -1), day 14, day 28 and day 35: total of four times. The experimenter(s) were blind regarding to the groups.

Rats were sacrificed via CO2 asphyxiation on Day 36. The knee articular structure was fixed in 4% buffered formalin solution for further histological analysis. Contralateral (non-injured) knees were also fixed in 4% buffered formalin solution.

Numerical results were given as means ± SD. Outliers data points (marked with asterisk) were identified following Grubbs' test analysis with alpha = 5% and were not included in the group average calculations. If applicable, statistical analysis was carried out using two-way (followed by Bonferroni post-hoc test) or one-way ANOVA (followed by Dunnett's Multiple Comparison post Test). A probability of 5% (p ≤ 0.05) was regarded as significant. In the figures, the degree of statistically significant differences between groups were illustrated as *p≤0.05, **p<0.01 and ***p<0.001.

### RESULTS

### Weight -bearing test

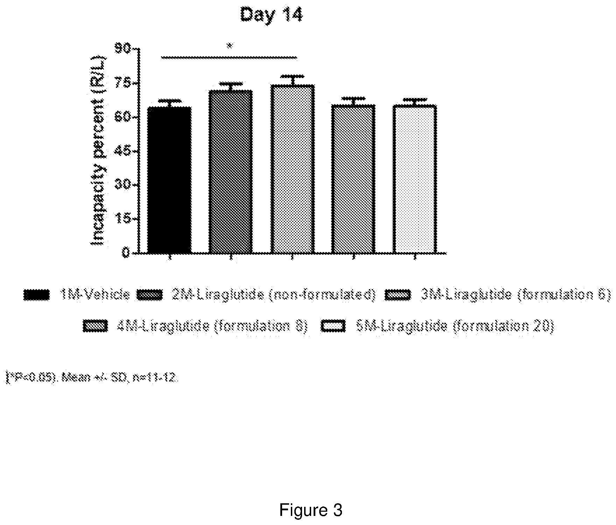

Weight-bearing changes in the rats with OA were assessed using an incapacitance meter that independently measures the weight that the animal distributes to each hind paw. All animals before OA induction distributed the weight equally on both hind paws. On day 14, a significant increase in weight bearing differences (R/L weight percent) was observed between control group and group 3M with a prolonged release of liraglutide (figure 3)

### Histology evaluation

The slides were examined by one pathologist who was blind to the treatment groups. Knees cross sections were evaluated for the following parameters:
Cartilage matrix loss width (0% cartilage is intact, 100% tidemark, 50% Midzone)
Cartilage degenerate score (score 0-5, see section 9.1).

Total cartilage degeneration width (0% cartilage is intact, 100% tidemark, 50% Midzone). This includes all possible degenerative changes.

Significant cartilage degeneration width. Measurement of more than 50% of the thickness is seriously compromised, (+/-).

Zonal depth ratio of lesions (microns).

Osteophytes (score 0-4, see section 9.2).

Calcified cartilage and subchondral bone damage score (score 0-5, see section 9.3).

Synovial reaction (score 0-4, see section 9.4).
Medial joint capsule repair (measurements in µm)
10. Growth of plate thickness (measurements in µm)

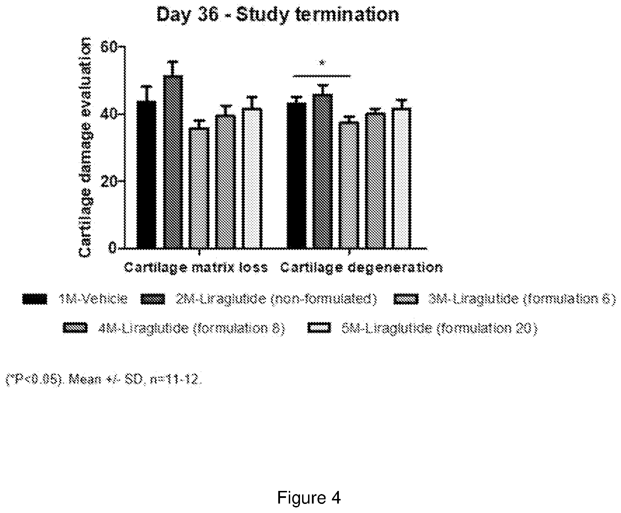

The Cartilage matrix loss width (%) was lowest in group 3M (35.8%) compared to vehicle treated control group1 M (43.6%). Total cartilage degeneration width (%): was also lowest in group 3M (37.5%) compared to vehicle treated control group1M (43.2%). Two-way ANOVA followed by Bonferroni post-hoc comparisons revealed statistically significant differences in Cartilage degeneration between control and Liraglutide treated animals group 3M as shown in figure 4. (*P<0.05). Mean +/- SD, n=11-12.

### CONCLUSION

The study objective was to evaluate 3 Liraglutide-based formulations efficacy utilizing a surgically-induced model of osteoarthritis in rats.

The results of this study show that Liraglutide in formulation 6 corresponding to high release of liraglutide (group 3M) induced a statistically significant decreased OA damage in weight bearing measurement by incapacitance meter compared to vehicle treated control group of animals on day 14 after OA induction. Von Frey test did not reveal statistically significant differences between all animal groups. Cartilage degeneration was also decreased in Liraglutide treated with formulation 6 group compared to control and other formulations. Weight bearing test and histology evaluation evidently were sensitive for the test of new therapeutically treatments in OA rat models.

Liraglutide formulated in formulation 6 has chondroprotective effect in vivo compared to the other formulations or non-formulated liraglutide.

### EXAMPLE 4 - DOSE-RESPONSE STUDY USING ALBUMIN-BASED FORMULATION OF LIRAGLUTIDE UTILIZING A SURGICALLY-INDUCED MODEL OF OSTEOARTHRITIS IN RATS

The principle of the test was based on the evaluation of albumin-formulated Liraglutide on disease parameters measurements in a rat OA model.

### MATERIALS AND METHODS

The test item is Liraglutide, the positive control is Dexamethasone (and non-formulated liraglutide Victoza^{®} from Novo Nordisk (Injectable solution at 6 mg/ml).

### FORMULATIONS

Vehicle (formulation excipient) consisted of albumin resuspended in phosphate buffer saline for intra-articular injection (25µl) to groups 1M and 7M.

Albumin-based formulations of Liraglutide (high, medium and low doses) were prepared as follow:
Liraglutide (supplied as powder) was dissolved in the appropriate volume of vehicle to reach 0.18 mg/kg (for groups 2M and 8M), 0.06 mg/kg (for group 3M) or 0.02 mg/kg (for group 4M) dose level in 25µl for intra-articular injection. Formulations were prepared on the day of the treatment for each of the three cycles.

The positive control that was used for group 5M is dexamethasone. The human clinical dose for knee treatment is 4 mg/injection which corresponds to 0.4 mg/kg for a rat. The dexamethasone injectable solution was supplied "ready-to-use" at a concentration of 4 mg/ml. The volume administered to the rats was 25-50µl depending on rat mean BW at each treatment day.

Non-formulated Liraglutide (Victoza^{®}) was supplied as a stock solution of 6 mg/ml. The human clinical "starting" dose for diabetic patients is 0.6 mg/day (supplied as repeated SC injections) which corresponds to 0.06 mg/kg for a rat. The stock solution of Victoza^{®®} was diluted 1000 times in injectable saline to reach the final concentration of 0.006 mg/ml for SC injections to group 6M (10 ml/kg).

### EXPERIMENTAL MODEL

Animals: Rats / Strain: SD
Gender: Males / number: 72 / Age: 6-8 weeks at study initiation
Source: Janvier Labs, France.
Initial Body weight: The average body weight was 260g at study initiation (on Day -1). The minimal and maximal weight recorded in each group was within the range of ± 20 % of the groups mean.
Diet: Animals were fed ad libitum a commercial rodent diet (Safe ref #A04). Animals had free access to filtered drinking osmotic water.
Contaminants: There were no contaminants in food and water supplies that had the potential to influence the outcomes of this test.

### EXPERIMENTAL DESIGN AND CONDITIONS

Study initiation and termination definition: OA induction day was defined as "day 1". For the main study, study termination was at "day 36". For the study with satellite groups, study termination was at "day 57".

Allocation to treatment groups: Rats were allocated randomly into one of the eight groups according to body weight.

Study Design and Time Line: The study was conducted in three cycles according to Table 1 for study design and Table 2 for study timeline.

### OA INDUCTION BY MEDIAL LIGAMENT TRANSECTION (MLT) PROCEDURE FOLLOWED BY RESECTION OF MEDIAL MENISCI

Anesthesia was induced in a chamber induction technique using inhalation anesthesia (Isoflurane at 5.0%). During surgery, the animal was maintained under Isoflurane at a level between 1.5 and 3.5% with air flow rate of 1-2 liters/minute. Ophthalmic ointment was applied on the eyes to prevent drying of the tissue during the anesthetic period. After induction of anesthesia, the right leg skin surface was clipped free of hair using electric animal clippers. After shaving the knee joint, the skin was disinfected with iodine and a para patellar skin incision was made on the medial side of the joint. An incision on the medial side of the patellar tendon provides access to the joint that was exposed; the medial ligament was transected, and the medial meniscus was resected using a microsurgical knife. The wound was closed with vicryl 5-0 suture. All operation procedures were performed using a surgical microscope. Group Allocation is show in Table 8 and study timeline in Table 9.

**Table 8. Group allocation.**

| Group | Animal ID | Treatment | OA induction | Dose level (mg/kg) | Dose volume | ROA |
|---|---|---|---|---|---|---|
| 1M (n=10) | 1,4,9,10,26, 31,35,42,53,57 | Vehicle (formulation excipient- albumin) | √ | - | 25µl | IA |
| 2M (n=10) | 2,5,12,16,25, 32,45,48,50,52 | Albumin-formulated Liraglutide high dose | √ | 0.18 | 25µl | IA |
| 3M (n=10) | 3,6,17,18,28, 33,38,46,54,55 | Albumin-formulated Liraglutide medium dose | √ | 0.06 | 25µl | IA |
| 4M (n=10) | 8,11,13,20,27, 39,40,43,49,51 | Albumin-formulated Liraglutide low dose | √ | 0.02 | 25µl | IA |
| 5M (n=10) | 7,15,19,21,29, 34,36,47,56,58 | Positive control (Dexamethasone 4 mg/ml) | √ | 0.40 | 25-50µl* | IA |
| 6M (n=10) | 14,22,23,24,30, 37,41,44,59,60 | Non-formulated Liraglutide (Victoza^{®}) | √ | 0.06 | 10 ml/kg | SC |
| 7M (n=6) | 61,62,63,64, 65,66 | Vehicle (formulation excipient- albumin) | √ | - | 25µl | IA |
| 8M (n=6) | 67,68,69,70, 71,72 | Albumin-formulated Liraglutide high dose | √ | 0.18 | 25µl | IA |

**Table 9. Study timeline.**

| **Study Day/week** | **Procedure** | **Remarks** |
|---|---|---|
| **Once a week** | Body weight | |
| **Once a week** | Knee diameters measurem | And with a measurement the day before and the day after surgery |
| | | (Not for groups 7M-8M) |
| | | Repeated IA administrations once a week |
| **Day -1** | Start of treatment | for 5 weeks (groups 1M-5M, 7M-8M) or 5 days a week SC injection for 2 weeks |
| | | (group 6M) |
| **Day 1** | OA induction | |
| **Days -1, 14, 28 a** | Weight bearing test | Not for groups 7M-8M |
| **Days 7 and 21** | Von Frey test | Not for groups 7M-8M |
| | | Bleeding and plasma separation |
| **Day 36** | Termination for groups 1M-6M | Right knee (diseased) collection and fixation |
| | | Left knee (healthy) collection and fixation |
| **Day 57** | Termination for groups 7M-8M | Right knee (diseased) collection and fixation |
| | | Left knee (healthy) collection and fixation |

### TEST AND EVALUATIONS

Joint swelling: Knee diameters measurement was performed to infer joint swelling as an indicator of inflammation. Measurement of the diameter of both knees was performed with a digital caliper upon anesthesia of the rats. Measurement was performed the day before surgery (for baseline), the day after surgery and then once a week until study termination. The experimenter(s) were blind regarding the groups.

Weight bearing test: Weight-bearing changes in the rats following OA induction was monitored using an incapacitance testing system. Postural imbalance, which reportedly indicates a change in the pain threshold and weight distribution of the limbs was followed. Each rat was placed so that each hind paw rests on a separate force plate on the incapacitance apparatus, and the weight borne by each hind limb was measured for 5 sec. The ratio of the weight borne by the right to left hind limb was calculated. The mean of three consecutive measurements for each rat was recorded. Weight bearing function (Incapacitance test) was performed on the animals at baseline (Day -1), day 14, day 28 and day 35: total of four times. The experimenter(s) were blind regarding the groups.

Animals sacrifice and tissue fixation: On day 36 (termination day for groups 1 M-6M), bleeding was performed on all animals. Rats were euthanized by a lethal dose of Euthasol vet. The knee articular structure was harvested and fixed in 4% buffered formalin solution for further histological analysis. Contralateral (non-injured) knees were also fixed in 4% buffered formalin solution. On day 57 (termination day for the satellite groups 7M-8M), rats were euthanized. The knee articular structure was harvested and fixed in 4% buffered formalin solution for further histological analysis. Contralateral (non-injured) knees were also fixed in 4% buffered formalin solution.

Histology analysis: Histology analysis was performed. Knee joint sections were stained with hematoxylin-eosin or toluidine blue staining to evaluate the extent of pathological lesions. Slides were scored as in N. Gerwin et al., Osteoarthritis and Cartilage 18 (2010) S24-S34.

Histological analysis was performed on:
Right knees (diseased): All groups as harvested on day 36, 10 animals/group. No. of samples: n=60
Left knees (healthy control): vehicle group 1M, 5 animals/group. No. samples: n=5
Right knees (diseased): all animals from groups 7M-8M as harvested on day 57, 6 animals/group. No. of samples: n=12
TOTAL no. samples: n=77
Statistical analysis: Numerical results were given as means ± Standard Deviation (SD). Outliers or excluded data points (marked with $) were not included in the group average calculations. If applicable, statistical analysis was carried out using two-way or one-way ANOVA (followed by Dunnett's Multiple Comparison post Test). A probability of 5 % (p ≤ 0.05) was regarded as significant. In the figures, results were given as means ± SEM and the degree of statistically significant differences between groups were illustrated as *p≤0.05, **p<0.01 and ***p<0.001.

### RESULTS

For the main study, six groups (1M-6M, n=9-10 per group) were followed from day -1 to day 36. Animal #2 from group 2M was excluded from the entire study because histological analysis revealed no lesion of the right knee articulation.

Knee measurements (KM): Knee measurements were recorded before surgery, the day after surgery and once a week thereafter. For each group, means of left and right knee diameter in the two dimensions were calculated.

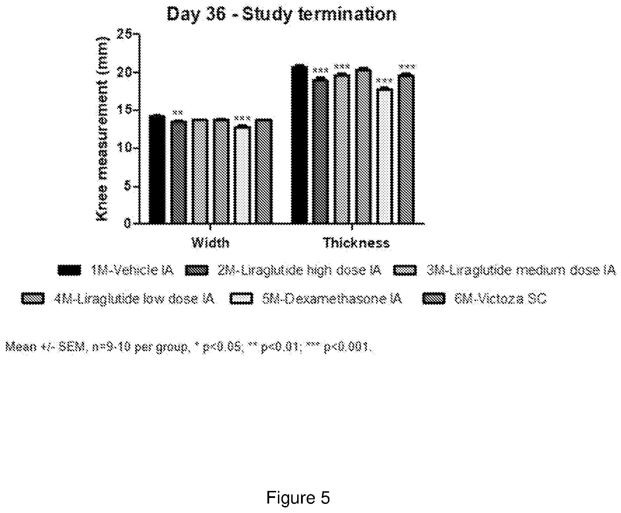

At termination, rats from groups 2M treated IA with Liraglutide in high dose (width and thickness), rats from groups 3M treated IA with Liraglutide in medium dose (thickness), a dose response is observed. Rats from group 6M treated SC with Victoza^{®} (thickness) had also significantly diminished knee measurements compared to vehicle-treated group 1M as shown in figure 5.

Weight bearing test: Weight-bearing changes in the rats with OA were assessed using the incapacitance meter that measures the weight that the animal distributes on each hind paw. Incapacitance test was performed on day -1 (baseline) and on days 14, 28 and 35. A significant increase in R/L ratio in % was observed for the group 6M treated with Victoza^{®} on day 14. This result, although non-significant, could be observed also during the other measurement periods (days 28 and 35). Slight non-significant trend in weight bearing differences was observed between control and Liraglutide IA treated animals with the medium dose (days 14 and 35 after OA induction) or the high dose (day 35).

### Histological analysis

The left hind limbs (from animals 1M1, 1M9, 1M26, 1M31 and 1M57) were provided as controls. As expected, knee articulations did not display any lesion.

In all the groups, the lesions that have been observed are from marked to severe. The typical observed pattern was a focally extensive tearing of the cartilage generally involving the whole medial tibial plate.

The margins of the ulceration were generally characterized by cartilaginous fibrillation) and/or necrosis with complete loss of the proteoglycan matrix. The subchondral bone was generally interlaced with large fibrocollagenous bundles (fibrosis). Just at the osteochondral interface (tidemark), involving more than the friction area, subchondral bone displayed necrosis (cells showing hyperacidophilia, caryorrexis, pycnosis with surrounding fibrin).

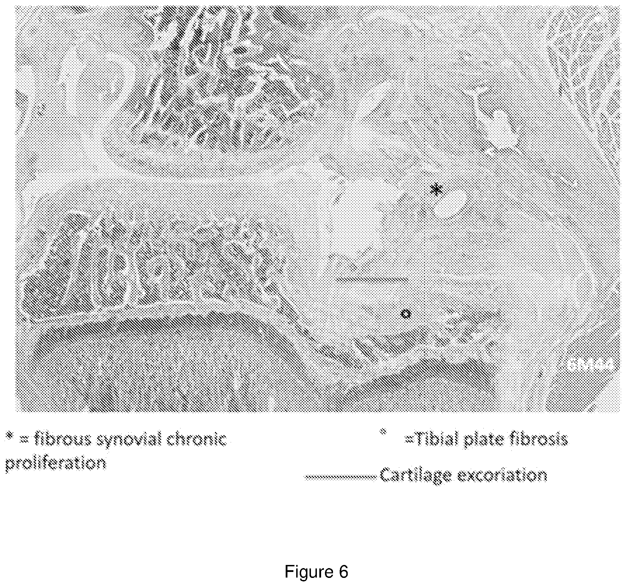

Dexamethasone IA treated group (Group 5M) and Victoza^{®} SC treated group (Group 6M) tend to show a lower cartilage loss. However, in some individual there was a subtotal replacement of the tibial plate by fibrotic tissue, forming synechia between medial meniscus remnants, femoral cartilage and synovial membrane as shown in figure 6.

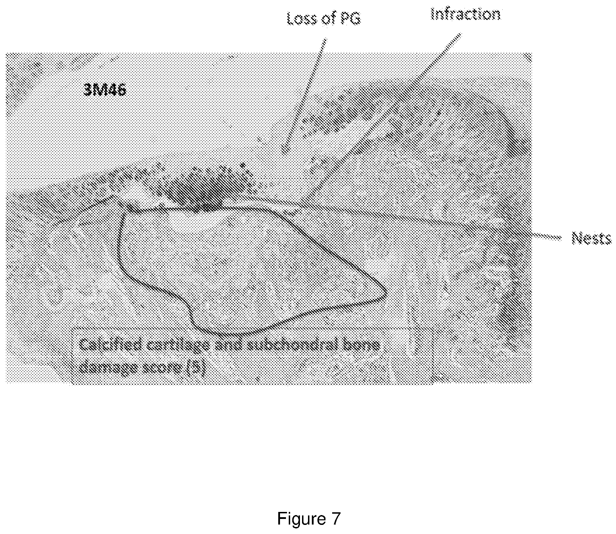

The repair changes observed were characterized by fibrocollagenous bundles within the synovial capsule which, in dexamethasone IA treated group (5M) and Victoza^{®®} SC treated group (6M), displayed large fibrous papillary highly vascularized projections sometimes forming synechia within all the articulation with other structures (medial meniscus remnants, articular cartilage, ligament remnants). In Liraglutide IA treated groups, no fibrosis was observed and no lesion differences: chondrocytes nests were observed within the cartilage in Liraglutide IA treated groups with a dose response pattern (Figure7): 3 animals/9 in high dose group, 2 animals/8 in medium dose group and 1 animal/9 in low dose group. In the Victoza^{®} SC treated group only 1 animal from 7 presented chondrocyte nests proliferation but not in the vehicle treated group (0 animal/10).

These observed chondrocytes proliferation suggests an attempt at cartilage regeneration.

### SATELLITE GROUPS STUDY

For the study with satellite groups, two groups (7M-8M, n=6 per group) were followed from day -1 to day 57. Histological parameters were measured as previously in the main study. Assessment of the percentage of recovery three weeks after the arrest of the treatment was analysed comparing Liraglutide high dose IA treated groups and vehicle groups.

**Table 10. Satellite Groups Study.**

| | | **1M** | **7M** | **% of Recovery Vehicle** | **2M** | **8M** | **% of Recovery Liraglutide IA** |
|---|---|---|---|---|---|---|---|
| **Cartilage matrix loss width** | **Surface** | 1996,80 | 1433,60 | 72% | 2016,33 | 856,33 | 42% |
| | **MidZone** | 1450,40 | 973,20 | 67% | 1837,67 | 525,17 | 29% |
| | **Tidemark** | 1497,00 | 771,80 | 52% | 1693,67 | 1007,83 | 60% |
| **Cartilage degeneration score** | **Z1** | 4,00 | 2,00 | 50% | 4,56 | 1,67 | 37% |
| | **Z2** | 4,70 | 4,00 | 85% | 4,89 | 4,17 | 85% |
| | **Z3** | 3,70 | 2,40 | 65% | 4,44 | 1,50 | 34% |
| | **Total** | 12,40 | 8,20 | 66% | 14,00 | 7,33 | 52% |
| **Total cartilage degeneration width** | | 2311,60 | 1493,40 | 65% | 2136,67 | 1809,17 | 85% |
| **Significant cartilage degeneration** | **Lesion thickness** | 157,90 | 119,60 | 76% | 247,67 | 134,17 | 54% |
| **Z1** | **Cartilage thickness** | 306,60 | 493,80 | 161% | 382,67 | 423,67 | 111% |
| | **Lesion thickness** | 369,90 | 402,60 | 109% | 425,56 | 347,83 | 82% |
| **Z2** | **Cartilage thickness** | 534,30 | 516,40 | 97% | 382,11 | 505,33 | 132% |
| | **Lesion thickness** | 164,50 | 149,40 | 91% | 325,22 | 151,33 | 47% |
| **Z3** | **Cartilage thickness** | 449,50 | 577,80 | 129% | 350,89 | 521,83 | 149% |
| **Osteophytes** | **Length** | 3,22 | 2,80 | 87% | 4,00 | 3,00 | 75% |
| **Calcified cartilage and subchondral bone damage score** | **Score (0-5)** | 4,80 | 4,20 | 88% | 4,44 | 3,67 | 83% |
| **Synovial membrane inflammation** | **Score (0-4)** | 3,50 | 3,00 | 86% | 3,11 | 3,17 | 102% |
| **Medial joint capsule repair** | | 476,60 | 424,80 | 89% | 316,13 | 721,50 | 228% |

Overall, the lesions that have been observed are marked for both groups. As observed during the main study, the typical observed pattern was a focally extensive tearing of the cartilage involving a large part of the medial tibial plate. The margins of the ulceration were characterized by cartilaginous fibrillation and/or necrosis with complete loss of the proteoglycan matrix.

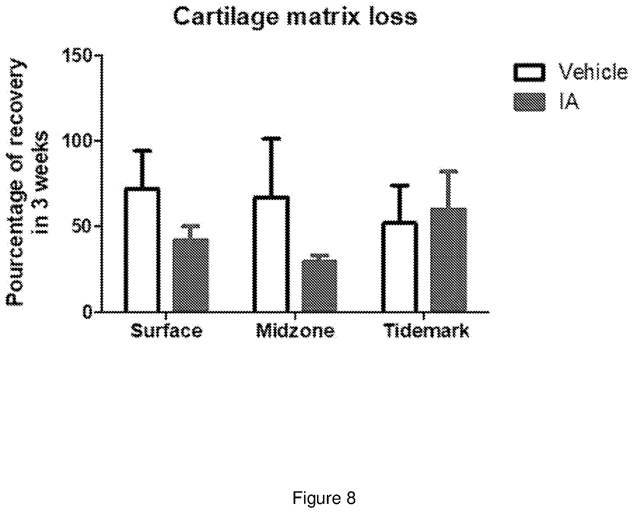

Animals treated IA with high dose Liraglutide (group 8M) tend to show a lower matrix loss compared to the vehicle-treated animals (group 7M) as well as a reduction of loss through the course of the study in the liraglutide IA treated group (Figure 8)

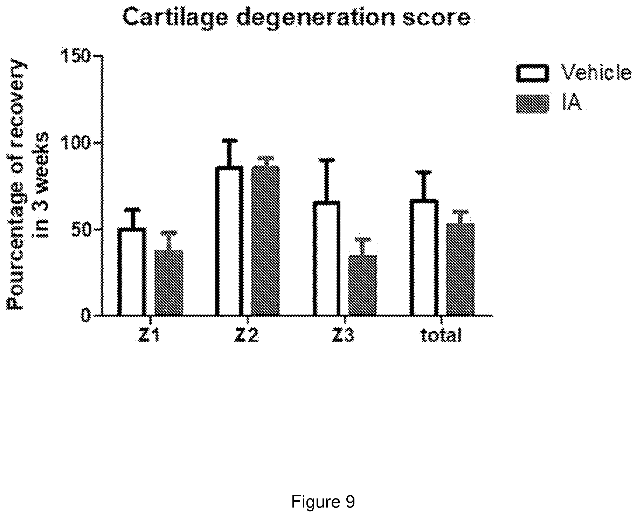

No differences were observed in degeneration score and total degeneration width. However, through the course of the study after the arrest of the treatment degeneration score was lower in liraglutide IA treated group in Z1 and Z3 (Figure 9).

There was no significant difference between osteophyte formation between the two groups. However, through the course of the study after the arrest of the treatment percentage of osteophyte was lower in tendency in liraglutide IA treated group.

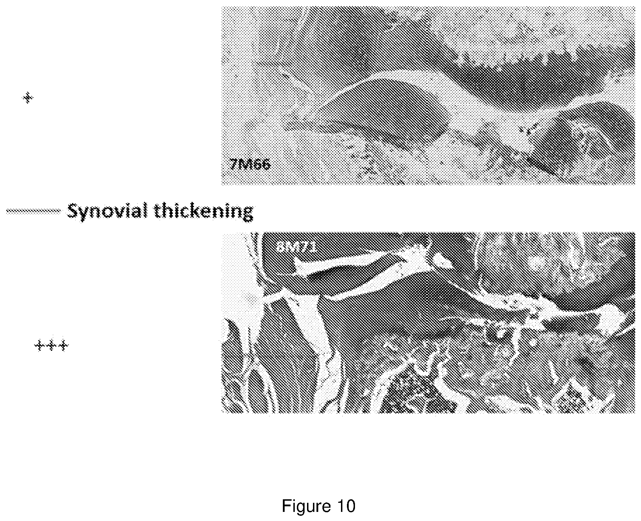

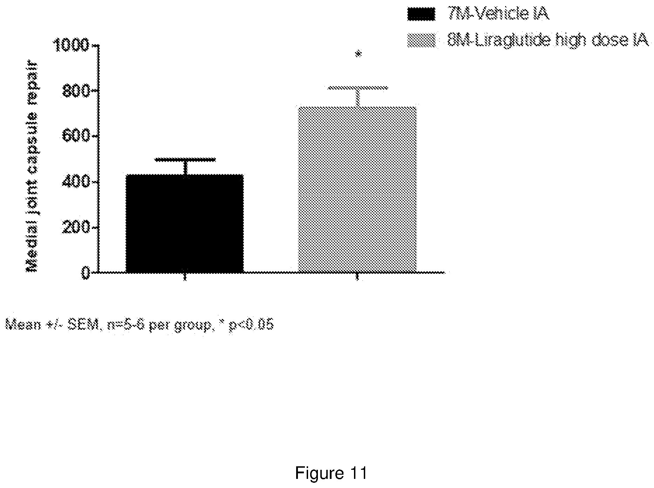

The high dose treated group exhibited a more important synovial repair observed by membrane thickening compared to control group (Figure 10 and 11).

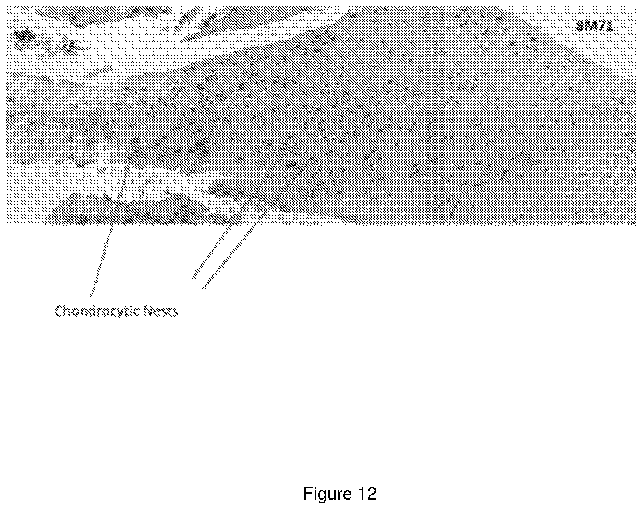

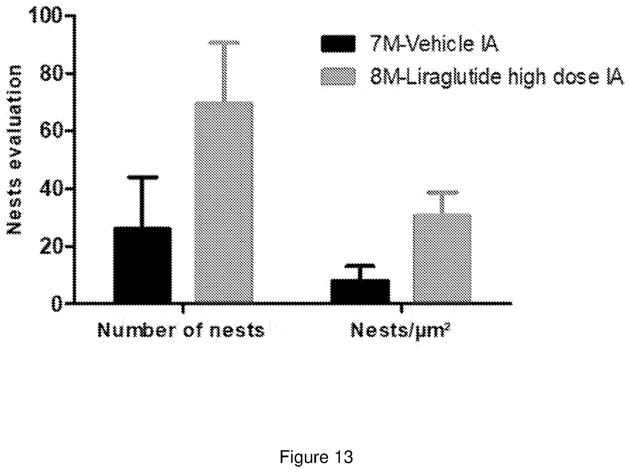

Examples of nests figures are presented in Figure 12. Significantly more chondrocytes nests were observed within the high dose test item treated group (5 animals/6; 30.6±7.0 nests/µm²) compared to the vehicle group (2 animals/5; 8.1±4.1 nests/µm²) as evaluated in figure 13.

### CONCLUSION

As previously observed during the main study, marked histological changes for each group concerning the cartilage, subchondral bone and synovial membrane were observed.

The synovial membrane repair changes are more intense in the IA treated group with high dose Liraglutide compared to vehicle group. Moreover, the cartilage degeneration changes were less marked in Liraglutide treated group compared with the vehicle group, but not statistically different.

Especially, more cartilage chondrocytic nests is observed in Liraglutide IA treated group demonstrating regeneration attempts within the cartilage.

Under study conditions, Dexamethasone IA and Victoza^{®®} SC treatments were able to mitigate several OA associated deficits (knee swelling, incapacitance, histology findings related to cartilage loss, however the repair was accompanied by fibrosis that was not observed in IA treated group.

Interestingly, histological analysis demonstrated chondrocyte nests within the cartilage for the groups treated with Liraglutide.

Those figures are indicative of chondrocytes proliferation demonstrating an attempt at cartilage repair. For the main study (Day 36), nests presence was not associated with synovial membrane repair changes in IA Liraglutide treated groups. However, for the satellite groups study (Day 57), chondrocytes nests found for the group of animals treated IA with Liraglutide in high dose, were associated with more marked synovial membrane repair changes (thickening).

Overall study results indicate that Liraglutide target relevant mechanism associated with inflammatory and regenerative processes relevant to OA.

### EXAMPLE 5: effect of Liraglutide on chondrogenesis in an in vitro DIFFERENTIATION model of human Mesenchymal Stem Cells

The inventors tested the effect of Liraglutide on chondrogenesis in an in vitro differentiation model of human Mesenchymal Stem Cells (hMSC) and evaluate whether or not Liraglutide promotes chondrogenesis.

### MATERIALS AND METHODS

**Test material:** Liraglutide.
**Test system:** human mesenchymal stem cells (StemPro BM, Cat A15652, ThermoFisher Scientific).
**Basal medium:** MesenPRO RS basal medium (ThermoFisher Scientific) supplemented with MesenPRO RS growth supplement, L-Glutamine (1%) and Gentamicin (10mg/ml, 50 µl for 100 ml of medium).
**Differentiation medium** (used as a positive control): StemPRO chondrogenesis differentiation medium (ThermoFisher Scientific) supplemented with StemPRO chondrogenesis differentiation supplement and Gentamicin (10mg/ml, 50 µl for 100 ml of medium)
**Procedures**
   Mesenchymal stem cells between 60-80% of confluence were used. The cells were detached from their support and a cell suspension at 1.6 x 10⁷ cells per milliliter in basal medium (MesenPRO RS basal medium + supplement) was prepared.

5 µl of this suspension were put into the well center of a 24-well plate. The plate was incubated for 2 hours in an incubator at 37°C with high humidity. After 2 hours, 1 ml of basal medium without (negative control) or with test item was added in each well according to Table 11 for study design and table 12 for study timeline. As a positive control, 1 ml of differentiation medium (StemPRO Chondrocyte Differentiation Basal Medium + Supplement) was used. The plate was put back into an incubator at 37°C + 5% CO₂ during 7, 14 or 21 days. During the differentiation phase, the medium was changed every 3-4 days.

**Table 11. Study design.**

| **Group** | **Treatment** | **Time** |
|---|---|---|
| 1 | Vehicle (PBS) in basal medium | 7, 14, 21 days of differentiation |
| 2 | Liraglutide 10 nM in basal medium | |
| 3 | Liraglutide 100 nM in basal medium | |
| 4 | Vehicle (PBS) in differentiation medium (positive control) | |

**Table 12. Study schedule.**

| **Procedure** | **Study day** | | | | | | | | | |
|---|---|---|---|---|---|---|---|---|---|---|
| | **1** | **4** | **8** | **9** | **10** | **15** | **16** | **17** | **22** | **23** |
| Plating and treating MSCs for differentiation into chondrocytes | √ | | | | | | | | | |
| Change medium | | √ | | | √ | | | √ | | |
| Study termination for alcian blue staining | | | √ | | | √ | | | √ | |

At termination of each study time (8, 15 or 22), plates were recovered for alcian blue staining and microscopy analysis. The medium was removed and 1 ml of PBS was added to gently rinse the cells. The PBS was removed, and 1 ml of Formaldehyde 4% was added for 30 minutes at room temperature.

Then, formaldehyde 4% was removed, and the fixed cells were gently rinse twice with 1 ml of distilled water. The distilled water was removed and 1 ml of Alcian Blue 1% (prepared in 0.1 N HCl) was added for 2 hours at room temperature, protect from light. The staining solution was removed and the cells were washed 2 or 3 times with 1 ml of 0.1 N HCl. The hydrochloric acid solution was removed and 1ml of distilled water was added to each well. The cells were observed under a microscope and pictures were taken.

### RESULTS

The effect of Liraglutide on sphere formation was evaluated by microscope observation 5 days per week. Moreover, Alcian blue staining was performed at three time points (e.g. after 7, 14 and 21 days of treatment). This dye incorporation reflects the presence of sulfated glycosaminoglycans (GAG) and confirms the formation of chondrocytes spheroids.

**Table 13. Summary table indicating the % of wells with spheres formation in the different conditions during the experiment.**

| | | | | | |
|---|---|---|---|---|---|
| **% of wells with alcian blue positive chondrocyte spheroids** | **Day** | **Basal medium with** | | | **Differentiation medium (Positive control)** |
| | | **Vehicle (PBS)** | **Liraglutide 10 nM** | **Liraglutide 100 nM** | |
| | 7 | 0 (0/18) | 33 (6/18) | 33 (6/18) | 39 (7/18) |
| | 14 | 0 (0/12) | 67 (8/12) | 58 (7/12) | 67 (8/12) |
| | 21 | 0 (0/6) | 67 (4/6) | 100 (6/6) | 83 (5/6) |

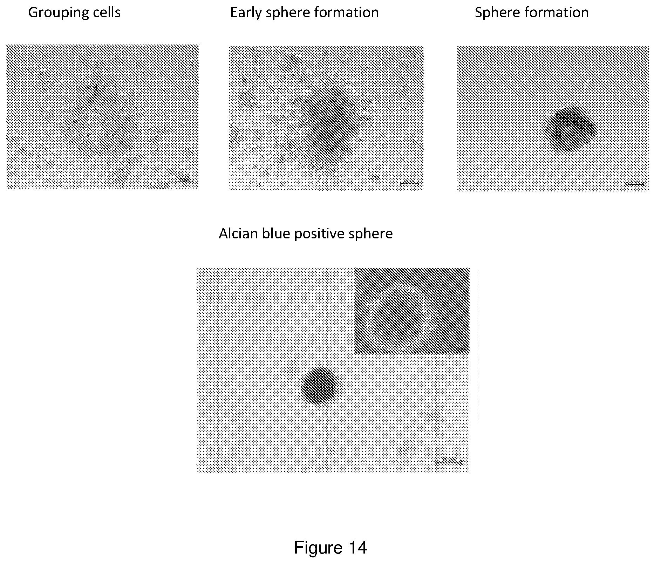

As shown in Table 13, no sphere formation was observed for vehicle-treated cells in basal medium during the study. Spheres formation was observed for the two tested doses of Liraglutide, with a dose-response. Indeed, for cells treated with 10 nM and 100nM Liraglutide, sphere formation was observed in 67% and 100% of treated wells on day 22, respectively. The differentiation medium contains all reagents required for inducing hMSC to be committed to the chondrogenesis pathway and generate chondrocytes. As expected, sphere formation was observed for vehicle-treated cells in this medium (83% of treated wells on day 22). Alcian blue coloration confirmed that the Liraglutide-induced spheres are chondrocytes spheroids. This result indicates that Liraglutide alone is able to induce hMSC to differentiate into chondrocytes. Example of sphere formation process and positive alcian blue staining is presented in Figure 14.

### CONCLUSION

In this study, we used an in vitro assay for chondrogenesis to test the effects of Liraglutide on this process.

In the presence of a basal medium, we demonstrated that Liraglutide induced the formation of spheres, with a dose response, while no sphere was observed for vehicle-treated cells. Chondrocytes spheres formation was confirmed by Alcian blue positive coloration (marker of cartilage matrix synthesis).

Under study conditions, our data indicate that Liraglutide alone induces hMSC to be committed to the chondrogenesis pathway and generates chondrocytes. This Liraglutide anabolic feature would allow to target the resident stem cell population in the articular region to stimulate cartilage repair via chondrocyte differentiation, which is considered as a promising approach for OA treatment.

### EXAMPLE 6: EFFECT OF LIRAGLUTIDE ON MURINE PRIMARY CHONDROCYTES VIABILITY

The objective of present study was to assess liraglutide effect on cell viability using murine primary chondrocytes.

### MATERIALS AND METHODS

Test material: Liraglutide
**Test system:** murine primary chondrocytes

### Formulation of medium for cell culture

2mM L-Glutamine, DMEM with 10% fetal bovine serum (FBS), 1% Penicillin/Streptomycin were used for cells culture from day 1 to day 7. At day 7, 2mM L-Glutamine, DMEM with 0.1% bovins serum albumin (BSA) and 1% Penicillin/Streptomycin (P/S) were used to work in FBS-free conditions.

### EXPERIMENTAL DESIGN AND CONDITIONS

### Study initiation definition

Day of plating cells in wells was considered as "Day 1" and study termination as "Day 9".

### Procedure

### Isolation of murine articular cartilage

Immature murine chondrocytes were derived from newborn mice (5-6 days old C57BI/6). This work was done in a sterile flow hood. After euthanizing mice by cutting the head with scissors, the animals were fixed in face-down position and the anterior legs were fixed with needles. The skin was removed on the hind limbs using scissors and pincer. The hind limbs were cut along the spine. The limbs were rid of their remains of skin and muscles. The paw was flattened with curved forceps, to release a small translucent and hard sphere, corresponding to femoral heads. When the sphere was isolated, it was placed in 30ml of 1X PBS. The rest of the paw was cleared of muscles and other tissues. The bone appeared in red-brown and the cartilage in white. The bone was cut on each side of the white part, it is the articulation (forming 2 spheres). The joint was cleaned from the surrounding tissue with a scalpel, then it was cut in half to separate the two spheres, and then cut in half again. This allows for easier digestion. Femoral condyles and tibial plateau were placed also in 30ml of 1X PBS.

### Isolation of immature murine chondrocytes

Pieces of cartilage were incubated twice in 10ml of digestion solution (DMEM, 2mM L-Glutamine + 1% P/S + Collagenase 3mg/ml) for 45min in incubator at 37°C with 5% CO₂ in a petri dish 100mm. Between the two digestions, pieces of cartilage were retrieved using 25 ml pipette and placed in a new petri dish. After the two digestions, a dispersion of the aggregates was made using 25ml pipette. Pieces of cartilage were incubated in 10ml DMEM, 2mM L-Glutamine + 1% P/S with collagenase D solution at 0.5mg/ml (diluted to 1/6) overnight in incubator at 37°C with 5% CO₂.

### Seeding of chondrocytes

After the overnight digestion, 10 ml of DMEM, 2mM L-Glutamine + 10% FBS were added to each petri dish to stop the collagenase D action. The medium and residual cartilage were retrieved and placed in a 50 ml Falcon tube. A dispersion of the aggregates was made using diminished sizes of pipettes to obtain a suspension of isolated cells which was filtered through a sterile 70µm cell strainer. Then, the cells were centrifuged for 10 min at 400g at 20°C. The medium was removed and the pellet was resuspended in 5ml of PBS to wash the cells. The cells were centrifuged for 10 min at 400g at 20°C, the PBS was removed and 15ml of DMEM 2mM L-Glutamine + 10% FBS + 1% P/S were added. The chondrocytes were counted in a Neubauer hemocytometer and observed to assess the viability of extracted cells. Chondrocytes were seeded at density of 40x10³ cells in 2 ml of DMEM 2mM L-Glutamine + 10% FBS + 1% P/S per well in 12-well plates. The culture was maintained under sterile conditions in incubator at 37°C with 5% CO₂.

### Culture of chondrocytes

Immature murine articular chondrocytes were confluent after 6-7 days. The medium culture was changed after 3 days of culture. At day 7, the DMEM medium containing 10% FBS was removed, the wells were rinsed twice with 1ml of PBS and 1ml of DMEM, 2mM L-Glutamine + 1% P/S + 0.1% BSA was added. At day 8, the medium was removed and treatment with 12 different concentrations of Liraglutide was performed in 500µl of DMEM, 2mM L-Glutamine + 1% P/S + 0.1% BSA per well (Table 14). The plates were incubated at 37°C + 5% CO₂ for 24 hours. Study timeline is presented in Table 15.

**Table 14. Study design.**

| **Group** | **Treatment** | **Time** |
|---|---|---|
| 1 | Vehicle (PBS) | 24 hours |
| 2 | Liraglutide 1.7nM | |
| 3 | Liraglutide 5.1nM | |
| 4 | Liraglutide 15.2nM | |
| 5 | Liraglutide 45.6nM | |
| 6 | Liraglutide 136.7nM | |
| 7 | Liraglutide 410nM | |
| 8 | Liraglutide 1.2µM | |
| 9 | Liraglutide 3.7µM | |
| 10 | Liraglutide 11.1µM | |
| 11 | Liraglutide 33.3µM | |
| 12 | Liraglutide 100µM | |
| 13 | Liraglutide 300µM | |
| 14 | Cells only (Blank) | |

Each condition treatment was run in triplicate.

**Table 15. Study schedule.**

| Procedure | Study day | | | | | |
|---|---|---|---|---|---|---|
| | Before study | 1 | 4 | 7 | 8 | 9 |
| Isolation of murine articular cartilage | √ | | | | | |
| Isolation of immature murine chondrocytes | √ | | | | | |
| Plating immature murine chondrocytes | | √ | | | | |
| Change medium | | | √ | | | |
| Change medium containing BSA | | | | √ | | |
| Treatment 24h with Liraglutide | | | | | √ | |
| Collect the supernatant | | | | | | √ |
| LDH Assay | | | | | | √ |

### Tests and evaluations

At study termination (Day 9), culture medium (± 500µl) of each well was collected in 1.5ml tube (1 tube per well), centrifuged at 4000rpm during 10min at room temperature and supernatant was put in a new 1.5ml tube. Samples were frozen at -70°C until the dosages were performed.

### LDH Assay

Lactate dehydrogenase secretion into culture medium was measured by LDH assay (Abcam). 100µl of supernatant were used for measurement of Lactate dehydrogenase levels secreted by damaged cells. LDH assay was performed according to procedure detailed in instructions for specific LDH Assay kit and was analyzed by Plate Reader (96-well) (Multiskan FC, Thermo Fisher). The wavelength to measure absorbance was 450 nm. The average optical density (OD) of read blank wells was subtracted from each reading.

### RESULTS

Lactate Dehydrogenase is a stable enzyme, present in all cell types and rapidly released into the cell culture medium upon damage of plasma membrane. The LDH enzyme has been detected using enzymatic coupling reaction and measured by Skanlt software for microplate reader, Thermo Fisher. LDH oxidizes lactate to generate NADH, which then reacts with WST substrate to generate yellow color. The intensity of color correlates directly with the cell number lysed. LDH activity has been quantified by spectrophotometer at OD₄₅₀ₙₘ. LDH Activity has been measured following 24h of incubation with 12 doses of liraglutide (1.7nM-300µM). A positive control was used, where 5µl of LDH enzyme was put directly in the wells. The % of cytotoxicity has been calculated by this formula: ((Test sample - Low control) / (High control - Low control)) x 100.

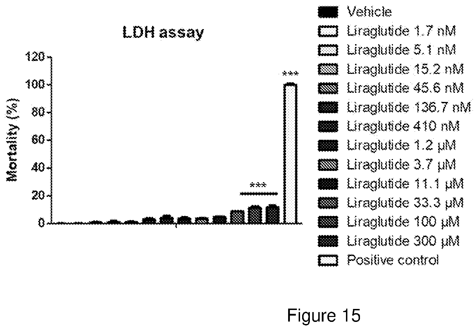

As shown in Figure 15, the presence of the lowest tested doses of Liraglutide (up to 11.1µM) induced the release of a small quantity of Lactate dehydrogenase in the medium.

However, there was no significant difference compared to vehicle-treated cells. In presence of the highest tested doses of Liraglutide (>30µM), the level of Lactate dehydrogenase enzyme detected increased significantly compared to vehicle, with a dose response. Indeed, the calculated % of mortality was: Vehicle: 0.0% ± 0.008 and Liraglutide 33.3µM: 8.5% ± 0.006; Liraglutide 100µM: 11.1% ± 0.051 and Liraglutide 300µM: 11.5% ± 0.069, p<0.001). The use of the positive control (marked in yellow) confirmed that all reagents of the kit are working properly.

### CONCLUSION

This study shows that depending on the liraglutide tested dose, mortality can be observed following 24h of incubation on chondrocytes.

### EXAMPLE 6: SOX9 EXPRESSION IN KNEE JOINT OF MONOIODOACETATE-INJECTED MICE FOLLOWING INTRA-ARTICULAR ADMINISTRATION OF ALBUMIN-based formulation OF liraglutide

SOX9 is a pivotal transcription factor in developing and adult cartilage. Its gene is expressed from the multipotent skeletal progenitor stage and is active throughout chondrocyte differentiation. While it is repressed in hypertrophic chondrocytes in cartilage growth plates, it remains expressed throughout life in permanent chondrocytes of healthy articular cartilage. SOX9 is required for chondrogenesis: it secures chondrocyte lineage commitment, promotes cell survival, and transcriptionally activates the genes for many cartilage-specific structural components and regulatory factors.

The objective of present study was to study SOX9 expression in knee joint of monoiodoacetate (MIA)-injected mice following intra-articular administration of albumin-based formulation of Liraglutide.

### MATERIALS AND METHODS

### Formulations

### Monoiodoacetate (MIA):

MIA as powder was resuspended in injectable saline to inject into knee joint 0.75mg in 5µl per mouse for groups 2M, 3M, 4M, 5M.

### Items formulation for treatment:

- Vehicle (formulation excipient) consisted of albumin human 5% resuspended in phosphate buffer saline (PBS) for intra-articular injection (5µl) to groups 1M and 2M.
- Albumin-based formulation of liraglutide:
   Liraglutide (supplied as powder) was dissolved in the appropriate volume of vehicle to inject into knee joint 10µg, 20µg or 30µg in 5µl per mouse for groups 3M, 4M and 5M, respectively.

### EXPERIMENTAL MODEL

Animals Species / Strain
Mice / C57BI/6
Gender / Age
Males / 12 weeks on day 1
Source
Janvier Labs, France.
Diet
Animals were fed ad libitum a commercial rodent diet (Safe ref #A04). Animals had free access to filtered drinking osmotic water.

### EXPERIMENTAL DESIGN AND CONDITIONS

### Study initiation definition

MIA induction day was defined in this study as "DAY 1" and study termination as "DAY 11". OA induction by intra-articular (IA) injection of MIA

Animals were anesthetized via a chamber induction technique using inhalation anesthesia (Isoflurane at 5%). During the procedure, the animals were maintained under Isoflurane at a level between 1.5 and 3% with an air flow rate of 1-2 liters/minute. The area surrounding the knee joint was wiped with alcohol. MIA was injected intra-articularly (IA) through the patellar tendon with 5µl containing 0.75mg. A 30-gauge, 0.5-inch needle that was fitted with cannulation tubing was used such that only 2 to 3 mm of the needle were allowed to puncture the joint. After injection, the knee was massaged to ensure even distribution of the solution. Animals were injected once on day 1 (groups 2M, 3M, 4M, 5M). For group 1M (sham control), 5µl of injectable saline were injected into knee joint.

### Study Design and Time Line

The study was conducted in three cycles according to Table 16 for study design and Table 17 for study timeline.

**Table 16. Group allocation.**

| **Group** | **N=** | **MIA induction (0.75mg in 5µl)** | **Treatment** | **Dose level** | **Dose volume** | **ROA** | **Dose regimen** |
|---|---|---|---|---|---|---|---|
| 1M | 6 | - | Vehicle (formulation excipient- albumin 5%) | - | 5µl | IA | Once on day 3 |
| 2M | 6 | √ | Vehicle (formulation excipient- albumin 5%) | - | | | |
| 3M | 6 | √ | Albumin-formulated Liraglutide | 10µg | | | |
| 4M | 6 | √ | | 20µg | | | |
| 5M | 6 | √ | | 30µg | | | |

Sham mice were allocated randomly to group 1M on day 1. For MIA injected mice, group allocation was performed on day 3 based on mice BW.

**Table 17. Study timeline.**

| **Study Day/week** | **Procedure** | **Remarks** |
|---|---|---|
| **twice a week** | Body weight | |
| **D1** | MIA injection, 0.75mg in 5µl, IA | Except for group 1M (sham control), 5µl injectable saline, IA |
| **D3** | Treatment (Vehicle or Liraglutide) | IA single injection, 5µl |
| **D11** | Termination | Right knee (diseased) collection for SOX9 RTqPCR analyses |

### TESTS AND EVALUATIONS

### Animals sacrifice and tissue collection

On day 11 (termination day), mice were euthanized. The knee articular structure (including synovium) was harvested and snap frozen into liquid nitrogen. RNA extraction was performed using SV total RNA isolation system kit (Promega) according to manufacturer's recommendations. RT-q-PCR analyses were performed for SOX9 marker. SOX9 is identified as the first transcription factor that is essential for chondrocyte differentiation and cartilage formation.

### RESULTS

### SOX9 RTqPCR analyses on knee articular structures

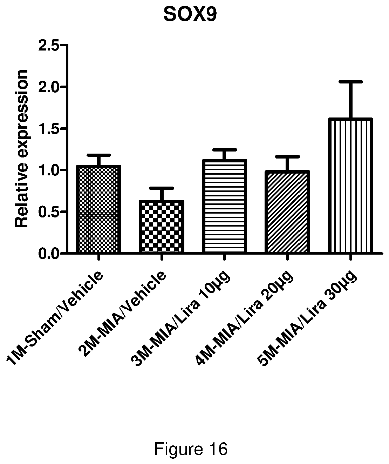

As presented in Figure 16, vehicle mice which received 0.75mg of MIA into knee joint on day 1 (group 2M) presented a decrease of 40% of SOX9 relative expression on day 11 compared to vehicle sham control (group 1M). When MIA-injected mice received an intra-articular injection of albumin-formulated liraglutide on day 3, the expression of SOX9 is restored and similar to the sham control group 1M for group 3M (Liraglutide 10µg) and 4M (Liraglutide 20µg). For the group 5M receiving 30µg of liraglutide, the relative expression of SOX9 is increased of 55% compared to sham control group 1M.

### CONCLUSION

The study objective was to perform RTqPCR for SOX9 into knee joint of monoiodoacetate-injected mice following intra-articular administration of formulated liraglutide.

The monoiodoacetate (MIA) model has become a standard for modelling joint disruption in osteoarthritis (OA) in both rats and mice. In this model, a single injection of MIA is delivered to the knee joint, which disrupts chondrocyte glycolysis by inhibiting glyceraldehyde-3-phosphatase dehydrogenase, inducing notably chondrocyte death. Chondrocytes, which differentiate following the condensation of mesenchymal stem cells, are responsible for the secretion of extracellular matrix molecules, such as collagens and proteoglycans. Transcription factor SOX9 is critical for chondrocyte differentiation and function. Using this animal model of OA, we showed that SOX9 expression is decreased following MIA injection, while intra-articular injection of formulated liraglutide restored or increased SOX9 relative expression compared to sham healthy controls.

This study shows therefore that locally-administered albumin-formulated Liraglutide targets relevant mechanisms associated with anabolism in a MIA-induced OA and inflammatory pain model in mice.

### EXAMPLE 7: EFFICACY STUDY of liraglutide ALPHA1-ACID GLYCOPROTEIN based-formulation UTILIZING collagenase type ii induced osteoarthritis model IN rats

The objective of present study was to perform an efficacy study using alpha1-acid glycoprotein-based formulation of Liraglutide utilizing a collagenase-induced model of osteoarthritis in rats.

### MATERIALS AND METHODS

### Formulations

### Collagenase type II:

Collagenase type II was dissolved in PBS in concentration of 20 000 U/ml to deliver 500U in 25µl.

Items formulation for treatment:
- Alpha1-acid glycoprotein (A1AGP) vehicle consisted of alpha1-acid glycoprotein 5% resuspended in PBS for intra-articular injection (25µl).
- Alpha1-acid glycoprotein-based formulation of liraglutide:
   Liraglutide (supplied as powder) was dissolved in the appropriate volume of alpha1-acid glycoprotein vehicle to reach 0.18 mg/kg dose level in 25µl for intra-articular injection.

### EXPERIMENTAL MODEL

Species / Strain
Rats / SD
Gender / Number / Age
Males / 20 / 6-7 weeks at study initiation
Source
Janvier Labs, France.

### Animal Management Housing

Animal handling was performed according to guidelines of the Federation of European Laboratory Animal Science Associations (FELASA). Animals were housed in plastic cages (2-3 per cage) with stainless steel top grill facilitating pelleted food and drinking water in plastic bottle; bedding: steam clean paddy husk (Safe) was used and bedding material was changed along with the cage at least once a week.

### Diet

Animals were fed ad libitum a commercial rodent diet (Safe ref #A04). Animals had free access to filtered drinking osmotic water.

### EXPERIMENTAL DESIGN AND CONDITIONS

### Study initiation definition

OA induction day was defined in this study as "DAY 1" and study termination as "DAY 43".

### OA induction by intra-articular (IA) injection of Collagenase type II

Animals were anesthetized via a chamber induction technique using inhalation anesthesia (Isoflurane at 5%). During the procedure, the animal was maintained under Isoflurane at a level between 1.5 and 3% with an air flow rate of 1-2 liters/minute. Collagenase type II was injected intra-articularly (IA) with 25 µl containing 500 U. Animals were injected twice: one injection on day 1 and a second one on day 4.

### Study Design and Time Line

The study was conducted according to Table 18 for study design and Table 19 for study timeline.

**Table 18. Group allocation.**

| **Group** | **N=** | **Treatment** | **OA induction** | **Dose level (mg/kg)** | **Dose volume** | **[C] (mg/ml )** | **RO A** | **Dose regime n** | **Equiva lent human dose per week** |
|---|---|---|---|---|---|---|---|---|---|
| 5M | 10 | Alpha1 -acid glycoprotein vehicle (alpha1-acid glycoprotein 5%) | √ | - | 25µl | - | IA | Once a week for 5 weeks (5 IA) | - |
| 6M | 9-10 | Alpha1 -acid glycoprotein-formulated Liraglutide | √ | 0.18 | 25µl | 2.3-3.3* | IA | | 1.8 mg |

| | | | | | | | | | |
|---|---|---|---|---|---|---|---|---|---|
| * was adapted in function of mean rat BW on the injection day | | | | | | | | | |

**Table 19. Study timeline.**

| **Study Day/week** | **Procedure** | **Remarks** |
|---|---|---|
| **Once a week** | Body weight | |
| **Day -1** | Start of treatment | Repeated IA administrations once a week for 5 weeks |
| **D1, D4** | Collagenase injection | 500 U in 25µl, IA |
| **Day 43** | Termination | Right knee (diseased) collection and fixation |
| | | Left knee (healthy) collection and fixation |

### TESTS AND EVALUATIONS

### Body weight

Body weight was recorded upon arrival, before study initiation and once a week thereafter.

### Animals sacrifice and tissue fixation

On day 43 (termination day), bleeding was performed on all animals. Rats were euthanized. The knee articular structure was harvested and fixed in 4% buffered formalin solution for further histological analysis. Contralateral (non-injured) knees were also fixed in 4% buffered formalin solution.

### Histology analysis

Rat knees immersed in buffered 3.7% formalin were delivered to a subcontractor for histology analysis. The histological analysis was performed by a veterinarian (DVM, DESV-Anatomic Pathology) who was blind to the group's treatment and the protocol during the whole analysis procedure. Knee joint sections were scored according to Osteoarthritis Cartilage. 2010 Oct;18 Suppl 3:S24-34.

### Statistical analysis

Numerical results were given as means ± Standard Deviation (SD). If applicable, statistical analysis was carried out using two-way or one-way ANOVA (followed by Dunnett's Multiple Comparison post Test) or t-test. A probability of 5 % (p ≤ 0.05) was regarded as significant. In the figures, results were given as means ± SEM and the degree of statistically significant differences between groups were illustrated as *p≤0.05, **p<0.01 and ***p<0.001.

### RESULTS

Results indicated a clear trend for the group 6M (intra-articular administration of Liraglutide in A1AGP vehicle) to display fewer cartilage lesions than the group 5M (A1AGP vehicle).

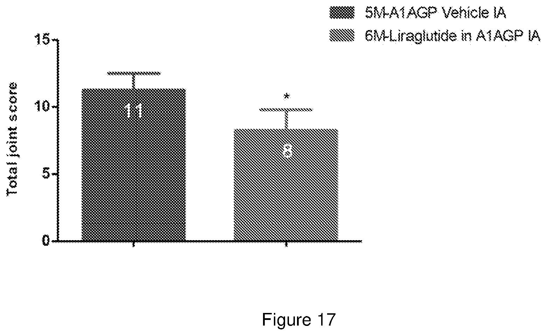

In support of this, a total joint score was calculated based upon the sum of the following sub section (de Visser et al, PLoS One. 2018 Apr 23;13(4):e0196308): cartilage matrix loss width (0-2), cartilage degeneration (0-5), cartilage degeneration width (0-4), osteophytes (0-4), calcified cartilage and subchondral bone damage (0-5) and synovial membrane inflammation (0-4). Figure 17 indicated a significant decrease of the total joint score for group 6M versus vehicle group 5M.

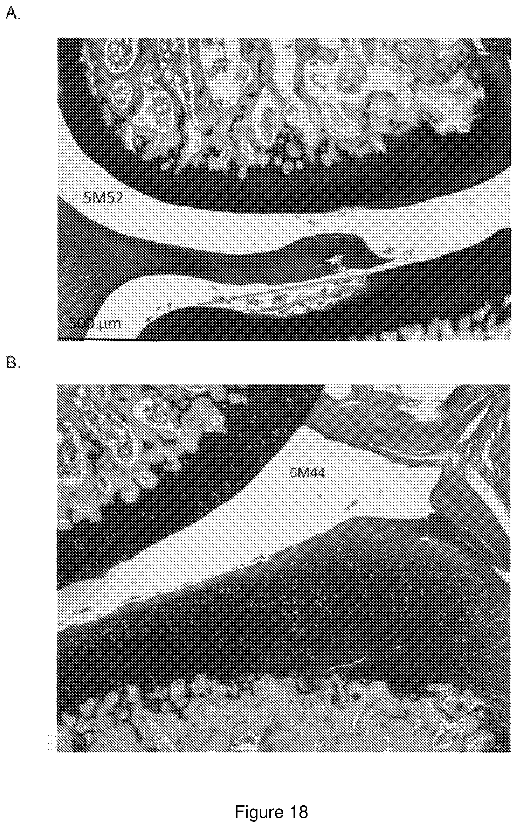

Representative pictures of right knee sections of animals from group 5M and 6M are shown on Figure 18.

### CONCLUSION

Histology findings indicated that Liraglutide IA tended to induce less cartilage loss and significantly diminished total joint score compared to vehicle, suggesting cartilage protection by local administration of Liraglutide.

Overall study results indicate that Liraglutide when administered locally is well tolerated and targeted relevant mechanisms associated with cartilage protection in this collagenase-induced OA model in rats.

## Claims

1. A composition for use in cartilage regeneration which comprises Glucagon Like Peptide-1 analogue, wherein, Glucagon Like Peptide-1 analogue is liraglutide.

2. A composition for use in cartilage regeneration according to claim 1, wherein, the concentration of said Glucagon Like Peptide-1 analogue is of about 0.1 nM to 625 µM**.**

3. A composition for use in cartilage regeneration according to claim 2, which further comprises at least 5% of more weight of a pharmaceutically acceptable formulation vehicle to be used in combination.

4. A composition for use in cartilage regeneration according to claim 3, wherein the pharmaceutically acceptable formulation vehicle is selected from the group consisting of albumin or alpha1-acid glycoprotein.

5. A composition for use in cartilage regeneration according to claim 4, wherein the pharmaceutically acceptable formulation vehicle concentration is about 0.1% to about 10% (wt/wt), preferably 5% (wt/wt), of the formulation.

6. A composition for use in cartilage regeneration according to claim 5, wherein the pharmaceutically acceptable formulation vehicle concentration is 5% (wt/wt) of the formulation.

7. A composition for use in cartilage regeneration according to claim 6, wherein the pharmaceutically acceptable formulation vehicle is albumin.

8. A composition for use in cartilage regeneration according to claim 6, wherein the pharmaceutically acceptable formulation vehicle is alpha1-acid glycoprotein.

## Patentansprüche

1. Zusammensetzung zur Verwendung bei der Knorpelregeneration, die ein Glucagonähnliches Peptid-1-Analog umfasst, wobei das Glucagon-ähnliche Peptid-1-Analog Liraglutid ist.

2. Zusammensetzung zur Verwendung bei der Knorpelregeneration nach Anspruch 1, wobei die Konzentration des Glucagon-ähnlichen Peptid-1-Analogs etwa 0,1 nM bis 625 µM beträgt.

3. Zusammensetzung zur Verwendung bei der Knorpelregeneration nach Anspruch 2, die weiter mindestens 5 % mehr Gewicht eines pharmazeutisch akzeptablen Formulierungsvehikels umfasst, das in Kombination verwendet werden soll.

4. Zusammensetzung zur Verwendung bei der Knorpelregeneration nach Anspruch 3, wobei das pharmazeutisch akzeptable Formulierungsvehikel ausgewählt ist aus der Gruppe, die aus Albumin oder saurem Alpha-1-Glykoprotein besteht.

5. Zusammensetzung zur Verwendung bei der Knorpelregeneration nach Anspruch 4, wobei die Konzentration des pharmazeutisch akzeptablen Formulierungsvehikels etwa 0,1 % bis etwa 10 % (w/w), vorzugsweise 5 % (w/w), der Formulierung beträgt.

6. Zusammensetzung zur Verwendung bei der Knorpelregeneration nach Anspruch 5, wobei die Konzentration des pharmazeutisch akzeptablen Formulierungsvehikels 5 % (w/w) der Formulierung beträgt.

7. Zusammensetzung zur Verwendung bei der Knorpelregeneration nach Anspruch 6, wobei das pharmazeutisch akzeptable Formulierungsvehikel Albumin ist.

8. Zusammensetzung zur Verwendung bei der Knorpelregeneration nach Anspruch 6, wobei das pharmazeutisch akzeptable Formulierungsvehikel saures Alpha-1-Glykoprotein ist.

## Revendications

1. Une composition pour son utilisation dans la régénération de cartilage qui comprend un analogue du Glucagon Like Peptide-1, dans laquelle l'analogue du Glucagon Like Peptide-1 est le liraglutide.

2. Une composition pour son utilisation dans la régénération de cartilage selon la revendication 1, dans laquelle la concentration dudit analogue du Glucagon Like Peptide-1 est d'environ 0,1 nM à 625 µM.

3. Une composition pour son utilisation dans la régénération de cartilage selon la revendication 2, qui comprend en outre au moins 5% en poids d'un véhicule pharmaceutiquement acceptable à être utilisé en combinaison.

4. Une composition pour son utilisation dans la régénération de cartilage selon la revendication 3, dans laquelle le véhicule pharmaceutiquement acceptable est choisi dans le groupe constitué par l'albumine et l'alpha-1-glycoprotéine acide.

5. Une composition pour son utilisation dans la régénération de cartilage selon la revendication 4, dans laquelle la concentration du véhicule pharmaceutiquement acceptable est d'environ 0,1% à environ 10% (m/m), de préférence 5% (m/m), de la formulation.

6. Une composition pour son utilisation dans la régénération de cartilage selon la revendication 5, dans laquelle la concentration du véhicule pharmaceutiquement acceptable est de 5% (m/m) de la formulation.

7. Une composition pour son utilisation dans la régénération de cartilage selon la revendication 6, dans laquelle le véhicule pharmaceutiquement acceptable est l'albumine.

8. Une composition pour son utilisation dans la régénération de cartilage selon la revendication 6, dans laquelle le véhicule pharmaceutiquement acceptable est l'alpha-1-glycoprotéine acide.
